# EUROPEAN PATENT SPECIFICATION

(11) **EP 4 060 338 B1**
(45) Date of publication and mention of the grant of the patent: **17.07.2024**
(21) Application number: 19953055.1
(22) Date of filing: 19.11.2019
(51) Int. Cl.: G01N 33/68, G01N 33/74, C07K 16/28, C07K 7/00

(54) **KIT AND METHOD FOR MIXED DETECTION OF PCT AND PRESEPSIN, AND USE THEREOF**
KIT UND VERFAHREN ZUR GEMISCHTEN DETEKTION VON PCT UND PRESEPSIN UND DESSEN VERWENDUNG
KIT ET PROCÉDÉ DE DÉTECTION MIXTE DE PCT ET DE PRÉSEPSINE ET LEUR UTILISATION

(43) Date of publication of application: 21.09.2022
(73) Proprietor: Shenzhen Mindray Bio-Medical Electronics Co., Ltd, Shenzhen, Guangdong 518057 (CN)
(72) Inventor: YU, Lina, Shenzhen, Guangdong 518057 (CN); LI, Ke, Shenzhen, Guangdong 518057 (CN); HE, Jianwen, Shenzhen, Guangdong 518057 (CN)
(74) Representative: KIPA AB
(86) International application number: PCT/CN2019/119537
(87) International publication number: WO 2021/097685

(56) References cited:
- EP-A1- 2 664 922
- EP-A1- 2 848 936
- EP-A1- 4 052 042
- WO-A1-2017/160599
- WO-A1-2018/114044
- WO-A1-2019/204634
- WO-A1-2021/083872
- CN-A- 1 711 283
- CN-A- 102 988 956
- CN-A- 103 733 068
- CN-A- 104 777 109
- CN-U- 208 060 540
- JP-B2- 4 040 666
- US-A1- 2013 157 291
- CARCAMO YAÑEZ VIVIANA ET AL: "Development and Validation of an Ultrasensitive Procalcitonin Sandwich Immunoassay", HIGH-THROUGHPUT, vol. 6, no. 18, 16 November 2017 (2017-11-16), pages 1 - 12, XP093015509, Retrieved from the Internet <URL:https://www.ncbi.nlm.nih.gov/pmc/articles/PMC5748597/pdf/high-throughput-06-00018.pdf> [retrieved on 20230119], DOI: 10.3390/ht6040018
- YUTAKA KONDO ET AL: "Diagnostic value of procalcitonin and presepsin for sepsis in critically ill adult patients: a systematic review and meta-analysis", JOURNAL OF INTENSIVE CARE, vol. 7, no. 1, 15 April 2019 (2019-04-15), XP055675465, DOI: 10.1186/s40560-019-0374-4
- FENG QIJIA ET AL: "Prediction of B-cell antigen epitope on CD14", SICHUAN MEDICAL JOURNAL, vol. 28, no. 9, 1 January 2007 (2007-01-01), pages 959 - 961, XP055814414, ISSN: 1004-0501, DOI: 10.16252/j.cnki.issn1004-0501-2007.09.003
- M. PLA-ROCA ET AL: "Antibody Colocalization Microarray: A Scalable Technology for Multiplex Protein Analysis in Complex Samples", MOLECULAR & CELLULAR PROTEOMICS, vol. 11, no. 4, 14 December 2011 (2011-12-14), XP055170250, ISSN: 1535-9476, DOI: 10.1074/mcp.M111.011460
- FORRESTER S ET AL: "Low-volume, high-throughput sandwich immunoassays for profiling plasma proteins in mice: Identification of early-stage systemic inflammation in a mouse model of intestinal cancer", MOLECULAR ONCOLOGY, ELSEVIER, vol. 1, no. 2, 1 September 2007 (2007-09-01), pages 216 - 225, XP022823846, ISSN: 1574-7891, [retrieved on 20070808], DOI: 10.1016/J.MOLONC.2007.06.001
- RASHWAN NAGWAN I.; HASSAN MOHAMMED H.; MOHEY EL-DEEN ZEINAB M.; AHMED AHMED EL-ABD: "Validity of biomarkers in screening for neonatal sepsis – A single center –hospital based study", PEDIATRICS & NEONATOLOGY, ELSEVIER, AMSTERDAM, NL, vol. 60, no. 2, 1 January 1900 (1900-01-01), AMSTERDAM, NL, pages 149 - 155, XP085656075, ISSN: 1875-9572, DOI: 10.1016/j.pedneo.2018.05.001

## Description

### TECHNICAL FIELD

The disclosure relates to a kit and a method for combined detection of procalcitonin (referred to as PCT for short) and a soluble CD14 subtype (referred to as sCD14-ST or Presepsin for short) in a sample to be tested, and the use thereof.

### BACKGROUND

Sepsis is a life-threatening organ dysfunction caused by a dysregulated host response to infection. It is reported in literatures that the incidence of sepsis is increasing year by year, and the fatality rate remains high, which has seriously threatened human life and health. According to the treatment guidelines for sepsis, early diagnosis is an effective way to improve the survival rate of patients with sepsis. Blood culture is the "gold standard" for diagnosis of sepsis; however, it has a low positive rate and a long culture time. Therefore, there is an urgent need to find a biomarker with high specificity and sensitivity for early diagnosis and accurate prediction of sepsis.

ISHIKURA, Hiroyasu *et al.* (EP 2 848 936 A1) discloses a method and the elements of a kits for the detection of procalcitonin and a soluble CD14 subtype in a sample per se or for the diagnosis of sepsis, prognosis or death risk assessment of a patient. MATSUYA Takeshi et al. (EP 2 664 922 A1) discloses a prediction method for the prognosis pf sepsis comprising measuring an Scd14-ST level in a sample. CARCAMO YANEZ VIVIANA et al., (2017), MDPI, vol. 6, no. 18, pages 1-12 discloses a method to detect PCT with a sandwich immunoassay. YUTAKA KONDO et al., (2019), JOURNAL OF INTENSIVE CARE, vol. 7, no. 1 discloses combined detection of PCT and Presepsin (sCD14-ST) is superior for the detection of sepsis than either alone. FENG QIJIA et al., (2007), SICHUAN MEDICAL JOURNAL, vol. 28, no. 9, pages 959-961 discloses a method to predict the B cell epitope for CD14 antigen. KIM IL HAN et al (US 2013/157291 A1) discloses a diagnostic marker for lung cancer comprising HpαR as an active ingredient. M. PLA-ROCA *et al.,* (2011), vol. 11, no. 4 discloses the use of a same label in multiplex sandwich assays. FORRESTER S *et al.,* (2007), vol. 1, no. 2, pages 216-225 discloses the use of a same label in multiplex sandwich assays. Rashwan Nagwan I et al., (1900), Pediatrics & Neonatology, vol. 60, no. 2, pages 149-155 discloses combined detection of PCT and Presepsin (sCD14-ST) is superior for the detection of sepsis than either alone.

### SUMMARY

The invention is defined in the claims.

Therefore, the disclosure provides a kit for combined detection of PCT and Presepsin in a sample to be detected and a method for combined detection of PCT and Presepsin in a sample to be detected. In addition, the disclosure also provides use of combined detection of PCT and Presepsin in a sample to be detected in the diagnosis and prognosis of sepsis in a patient.

A first aspect of the disclosure relates to a kit for combined detection of procalcitonin and a soluble CD14 subtype in a sample to be tested, the kit comprising: a capture antibody mixture, the capture antibody mixture comprising a procalcitonin capture antibody coated on a solid phase and a soluble CD14 subtype capture antibody coated on a solid phase; and a detection antibody mixture, the detection antibody mixture comprising a labeled procalcitonin detection antibody and a labeled soluble CD14 subtype detection antibody;
wherein the procalcitonin detection antibody and the soluble CD14 subtype detection antibody are labeled with the same label.

A second aspect of the disclosure relates to a method for combined detection of procalcitonin and a soluble CD14 subtype in a sample to be tested, the method comprising:
mixing a sample to be tested, a procalcitonin capture antibody coated on a solid phase, and a soluble CD14 subtype capture antibody coated on a solid phase, so that the procalcitonin capture antibody coated on the solid phase binds to the procalcitonin in the sample to be tested, and the soluble CD14 subtype capture antibody coated on the solid phase binds to the soluble CD14 subtype in the sample to be tested;
adding a labeled procalcitonin detection antibody and a labeled soluble CD14 subtype detection antibody to the mixture, so that the labeled procalcitonin detection antibody binds to the procalcitonin bound on the procalcitonin capture antibody to form a sandwich complex, and the labeled soluble CD14 subtype detection antibody binds to the soluble CD14 subtype bound on the soluble CD14 subtype capture antibody to form a sandwich complex;
washing the sandwich complexes to remove unbound substances;
adding a detection substrate to the washed sandwich complexes to detect the combined concentration level of the procalcitonin and the soluble CD14 subtype in the sample to be tested;
wherein the procalcitonin detection antibody and the soluble CD14 subtype detection antibody has the same label, wherein the label is an enzyme, and the detection substrate is a substrate for the enzyme.

A third aspect of the disclosure relates to use of a soluble CD14 subtype antibody and a procalcitonin antibody in the preparation of an analysis reagent for identifying whether a patient suffers from sepsis, where a sample to be tested from the patient, a procalcitonin capture antibody coated on a solid phase and a soluble CD14 subtype capture antibody coated on a solid phase are mixed, and then a labeled procalcitonin detection antibody and a labeled soluble CD 14 subtype detection antibody are added to detect the combined concentration level of the procalcitonin and the soluble CD14 subtype in the sample to be tested, and an increase in the combined concentration level relative to a reference value is associated with an increase in possibility of the patient suffering from sepsis;
wherein the procalcitonin detection antibody and the soluble CD14 subtype detection antibody are labeled with the same label.

A fourth aspect of the disclosure relates to use of a soluble CD 14 subtype antibody and a procalcitonin antibody in the preparation of an analysis reagent for evaluating the prognosis of a suspected sepsis patient, wherein a sample to be tested from the patient, a procalcitonin capture antibody coated on a solid phase and a soluble CD14 subtype capture antibody coated on a solid phase are mixed, and then a labeled procalcitonin detection antibody and a labeled soluble CD 14 subtype detection antibody are added to detect the combined concentration level of the procalcitonin and the soluble CD14 subtype in the sample to be tested, and the increase in the combined concentration level relative to a reference value is associated with an increase in risk of death in the suspected sepsis patient;
wherein the procalcitonin detection antibody and the soluble CD14 subtype detection antibody are labeled with the same label.

The kit, method and use provided in the embodiments of the disclosure may detect a combined signal value of PCT and Presepsin in a sample in a reaction system, the comparison between the combined signal value and a reference value may be used for evaluating the possibility of a patient suffering from sepsis and evaluating the prognosis of a suspected sepsis patient, and comparing to respectively detecting PCT or Presepsin in a sample, the method has a more efficient diagnosis and a better prognostic power. In addition, for combined detection of PCT and Presepsin in a sample, only one detection is required, so that the economic cost and time cost of the sample testing may be reduced, the blood consumption may be decreased, and also the effect of reagents of different production batches on the detection results may be reduced, which is conducive to reagent production and instrument automatization.

### BRIEF DESCRIPTION OF THE DRAWINGS

Preferred embodiments of the disclosure are described below with reference to the accompanying drawings. The objectives and advantages of the disclosure will be further understood from the following detailed description and accompanying drawings. The accompanying drawings show the following:
FIG. 1 shows a graph of ROC curve for evaluating whether a patient suffers from sepsis which is obtained by mixing a PCT capture antibody and a Presepsin capture antibody and mixing a PCT detection antibody and a Presepsin detection antibody at different ratios.
FIG. 2 shows a graph of ROC curve for evaluating whether a patient suffers from sepsis according to the embodiments of the disclosure.
FIG. 3 shows a graph of ROC curve of detection of PCT alone, detection of Presepsin alone, and combined detection of PCT and Presepsin for evaluating whether a patient suffers from sepsis, respectively.
FIG. 4 shows a graph of survival probability curve for evaluating the prognosis of sepsis according to the embodiments of the disclosure.
FIG. 5 shows a graph of ROC curve of detection of PCT alone, detection of Presepsin alone, and combined detection of PCT and Presepsin for evaluating the prognosis of sepsis, respectively.
FIG. 6 shows SDS-PAGE and Western Blot for a prokaryotic expression protein.
FIG. 7 shows SDS-PAGE and Western Blot for a eukaryotic expression protein.
FIG. 8 shows SDS-PAGE and Western Blot for an anti-soluble CD14 subtype polyclonal antibody.

### DESCRIPTION OF THE PREFERRED EMBODIMENTS

The disclosure will be described below in more detail by way of specific embodiments. However, these embodiments are only representative and the disclosure should in no way be construed as being limited to these embodiments.

Currently, in the diagnosis of sepsis, clinically widely used biomarkers are interleukin 6 (IL-6), C reactive protein (CRP) and procalcitonin (PCT). CRP is an acute phase protein synthesized by the liver. It has strong sensitivity to bacterial infection, but has low specificity, long half-life and no clear correlation with the progression of sepsis. The peak values of IL-6 appear early and can rise before clinical symptoms appear, which is beneficial for early diagnosis of sepsis, but has low sensitivity. PCT is widely used in the diagnosis of infection and sepsis. PCT is often not elevated in nonbacterial infections. PCT is often not significantly elevated in certain types of infections, such as skin and soft tissue infections. PCT is not suitable for determining the prognosis of septic shock infection in perioperative abdominal infections. In addition, in some non-infectious diseases, there is also an elevation in PCT. Although PCT has been shown to have a clear correlation with infection, false-positive diagnoses are prone to occur in some cases such as trauma, surgery, and burns. Therefore, PCT is not a perfect marker for the diagnosis of infection and sepsis, and PCT alone is not reliable for the diagnosis of infection and sepsis.

Soluble CD14 subtype (referred to as sCD14-ST or Presepsin for short), as a new biological indicator of sepsis, has potential application value in the diagnosis and prognosis of sepsis and in the guidance of the use of antimicrobial drugs in sepsis. Presepsin is an N-terminal fragment of sCD14 after being cleaved by proteases such as cathepsin D in the plasma, and has a relative molecular weight of about 13 kDa. Shirakawa et al. established an endotoxic shock model and a cecal ligation and perforation model (CLP model) in rabbits and found that the former did not result in an elevation in the concentration of Presepsin, whereas the CLP model resulted in a significant elevation. Therefore, it is believed that the elevation of Presepsin is resulted from phagocytosis caused by bacterial infection, rather than by physiological resistance to inflammation. At present, Presepsin is considered to have high specificity in evaluating the severity and prognosis of sepsis, and can accurately guide the use of antimicrobial drugs in sepsis. Nonetheless, Presepsin remains deficient as a diagnostic label in certain infection scenarios.

Therefore, provided in the embodiments of the disclosure are a kit and a method for combined detection of PCT and Presepsin in a sample to be tested with a more efficient diagnosis and a better prognostic capability, and the use thereof.

In the context of the disclosure, when these expressions, characteristics, values or ranges are referred to in conjunction with expressions such as "about, substantially, generally, at least, at a minimum", the disclosure also includes the exact or precise expressions, characteristics, values or ranges.

In the disclosure, the term "detection" may be used interchangeably with the terms such as "determination", "quantification" and "analysis", and is meant to encompass both quantitative and qualitative determinations. The test in the disclosure is performed in vitro.

In the scope of the disclosure, the term ROC (receiver operating characteristic) curve refers to a curve obtained by dividing the diagnostic test results into several critical points, using the sensitivity corresponding to each critical point as the ordinate and the specificity as the abscissa, and plotting. The ROC curve is an effective tool for comprehensive and accurate evaluation of diagnostic tests. Another effect of the ROC curve is to determine the optimal threshold for detection. In most cases when a critical point is determined by an ROC curve method, a point as close to the upper left as possible on the curve is selected, and is determined as the optimal critical point in combination with the professional situation. In the application, according to the ROC curve, combined with the sensitivity and specificity results of each point of tangency, a point of tangency with the largest Youden index as close to the upper left as possible on the curve is selected as the optimal critical point, so that the sensitivity and specificity of the test are high, and the misdiagnosis rate and missed diagnosis rate are low.

In the disclosure, the kit for combined detection of PCT and Presepsin in a sample to be tested is also referred to as a "combined detection kit".

The first aspect of the disclosure provides a kit (a combined detection kit) for combined detection of PCT and Presepsin in a sample to be tested, comprising: a solid phase component, the solid phase component including a procalcitonin capture antibody (a PCT capture antibody) coated on a solid phase and a soluble CD14 subtype capture antibody (a Presepsin capture antibody) coated on a solid phase; and a labeling component, the labeling component including a labeled procalcitonin detection antibody (a PCT detection antibody) and a labeled soluble CD14 subtype detection antibody (a Presepsin detection antibody); wherein the procalcitonin detection antibody and the soluble CD14 subtype detection antibody are labeled with a same label.

In some embodiments of the first aspect of the disclosure, the solid phase component is present in the kit in the form of a capture antibody mixture, where the capture antibody mixture is mixed with a procalcitonin capture antibody coated on a solid phase and a soluble CD14 subtype capture antibody coated on a solid phase. In other embodiments, the solid phase component may also include a procalcitonin capture antibody component coated on a solid phase and a soluble CD14 subtype capture antibody component coated on a solid phase that are separately packaged from each other.

Similarly, in some embodiments of the first aspect of the disclosure, the labeling component is present in the kit in the form of a detection antibody mixture, where the detection antibody mixture is mixed with a labeled procalcitonin detection antibody and a labeled soluble CD14 subtype detection antibody. In other embodiments, the labeling component may also include a labeled procalcitonin detection antibody and a labeled soluble CD14 subtype detection antibody that are separately packaged from each other.

In some embodiments of the first aspect of the disclosure, the procalcitonin capture antibody and the soluble CD14 subtype capture antibody are separately coated on different solid phases. Certainly, the procalcitonin capture antibody and the soluble CD14 subtype capture antibody may also be co-coated on the same solid phase.

In some embodiments of the first aspect of the disclosure, the mixing ratio of a PCT capture antibody to a Presepsin capture antibody is determined according to the principle of maximizing the diagnostic efficacy for diagnosing sepsis, that is, the mixing ratio is selected according to the principle of maximizing the AUC of the ROC curve. The mixing ratio of a PCT capture antibody to a Presepsin capture antibody in a solid phase component may be in the range of about 5: 1 to about 1: 5, preferably in the range of about 3: 1 to about 1:5, more preferably in the range of about 3: 1 to 1:3, in particular about 3: 1 or 1: 1 or 1: 3, and more particularly preferably about 1:3.

In some embodiments of the first aspect of the disclosure, the mixing ratio of a PCT detection antibody to a Presepsin detection antibody is determined according to the principle of maximizing the diagnostic efficacy for diagnosing sepsis, that is, the mixing ratio is selected according to the principle of maximizing the AUC of the ROC curve. The mixing ratio of a PCT detection antibody to a Presepsin detection antibody in a labeling component may be in the range of about 5: 1 to about 1:5, preferably in the range of about 3: 1 to about 1:5, more preferably in the range of about 3: 1 to 1: 3, in particular about 3: 1 or 1: 1 or 1: 3, and more particularly preferably about 1:3.

In some embodiments of the first aspect of the disclosure, the mixing ratio of a PCT capture antibody to a Presepsin capture antibody in a solid phase component may be equal to the mixing ratio of a PCT detection antibody to a Presepsin detection antibody in a labeling component, and the mixing ratios are preferably 1: 3.

In some embodiments of the first aspect of the disclosure, both a PCT capture antibody and a PCT detection antibody are commercially available, where the PCT capture antibody is, for example, Anti-hPCT 4003 SPTN-5 (Medix Biochemica), and the PCT detection antibody is, for example, Anti-hPCT 4051 SPTN-5 (Medix Biochemica).

In some embodiments of the first aspect of the disclosure, the soluble CD14 subtype capture antibody specifically recognizes an epitope composed of the amino acid sequence of SEQ ID No.42, and the soluble CD14 subtype capture antibody the soluble CD14 subtype capture antibody comprises:
(i) heavy chain variable region (VH) complementarity determining regions (CDRs):
   VH CDR1: X₁X₂X₃MX₄;
   VH CDR2: YIX₅X₆ADX₇;
   VH CDR3: X₈X₉X₁₀AX₁₁;
      and
(ii) light chain variable region (VL) complementarity determining regions (CDRs):
   VL CDR1: KX₁₂X₁₃X₁₄N;
   VL CDR2: LX₁₅X₁₆;
   VL CDR3: VX₁₇X₁₈X₁₉;
   where X₁ to X₁₉ are one or more of the amino acid sequences listed below as options:
      Xi=any one of the amino acids;
      X₂=F or V; X₃=A, E or K; X₄=A or L;
      X₅=SYMGS, SSGSS, AYTMY, TYSGS or SSKSS;
      X₆=GAYY, TIYY, AKYY, TKAS or SNLA;
      X₇=AKLG, TVKG, SYTA, MTKG or YGNT;
      X₈=A or Q; X₉=G or Q; X₁₀=Q or F; X₁₁=M, Y or V;
      X₁₂=YYAS, SSAT, SSQS or SGSS;
      X₁₃=AAKL, LLAT, LLYS or KAAS;
      X₁₄=YRNIKL, TWAKGN, NGKTYL or YTNGAL;
      X₁₅=VQS, KTS, QTS or VSK;
      X₁₆=LAS, LDS, LTA or DSK;
      X₁₇=G, A, S or Q;
      X₁₈=GTH, GNT, GTA or TAI;
      X₁₉=FITA, FPRT, YGHV or NYGH.

In some embodiments of the first aspect of the disclosure, the soluble CD14 subtype capture antibody comprises VH CDR1, VH CDR2 and VH CDR3, as well as VL CDR1, VL CDR2 and VL CDR3, where VH CDR1, VH CDR2 and VH CDR3, as well as VL CDR1, VL CDR2 and VL CDR3 are selected from the amino acid sequences described in Table 1 (see Table 1).

**Table 1**

| | SEQ ID NO | Amino acid sequences |
|---|---|---|
| VH CDR1 | 1 | SVAML |
| | 2 | SVEML |
| | 3 | SFAML |
| | 4 | SFAMA |
| | 5 | SVKML |
| | 6 | SFKMA |
| VH CDR2 | 7 | YISYMGSTIYYADAKLG |
| | 8 | YISSKSSSNLAADAKLG |
| | 9 | YISSGSSTIYYADTVKG |
| | 10 | YISSGSSTIYYADMTKG |
| | 11 | YITYSGSGAYYADYGNT |
| | 12 | YISYMGSTIYYADTVKG |
| | 13 | YISSKSSTIYYADTVKG |
| VH CDR3 | 14 | AGFAY |
| | 15 | QQFAY |
| | 16 | QGFAY |
| | 17 | QGFAM |
| | 18 | QQVAY |
| | 19 | AGFAM |
| | 20 | AGFAY |
| VL CDR1 | 21 | KSSQSLLYSNGKTYLN |
| | 22 | KYYASAAKLYRNIKLN |
| | 23 | KYYASLLATYRNIKLN |
| | 24 | KYYASLLATTWAKGNN |
| | 25 | KSSQSLLYSTWAKGNN |
| | 26 | KSSQSLLYSYRNIKLN |
| VL CDR2 | 27 | LVQSLDS |
| | 28 | LVSKLTA |
| | 29 | LVSKLDS |
| | 30 | LVQSDSK |
| | 31 | LQTSDSK |
| | 32 | LVSKLAS |
| VL CDR3 | 33 | VQGTHNYGH |
| | 34 | VGGTHFPRT |
| | 35 | VGTAIFPRT |
| | 36 | VGGTHFITA |
| | 37 | VSGTHFPRT |
| | 38 | VQGTHFPRT |
| | 39 | VQTAIFPRT |
| | 40 | VQGTHYGHV |
| | 41 | VQGNTFITA |

In some embodiments of the first aspect of the disclosure, the soluble CD14 subtype capture antibody comprises VH CDR1, VH CDR2 and VH CDR3, as well as VL CDR1, VL CDR2 and VL CDR3, where VH CDR1, VH CDR2 and VH CDR3, as well as VL CDR1, VL CDR2 and VL CDR3 are selected from the amino acid sequences described in Table 2 (see Table 2).

**Table 2**

| | SEQ ID NO | Amino acid sequences |
|---|---|---|
| VH CDR1 | 1 | SVAML |
| | 2 | SVEML |
| | 3 | SFAML |
| | 4 | SFAMA |
| | 5 | SVKML |
| | 6 | SFKMA |
| VH CDR2 | 7 | YISYMGSTIYYADAKLG |
| | 8 | YISSKSSSNLAADAKLG |
| | 9 | YISSGSSTIYYADTVKG |
| | 10 | YISSGSSTIYYADMTKG |
| VH CDR3 | 14 | AGFAY |
| | 15 | QQFAY |
| | 16 | QGFAY |
| | 17 | QGFAM |
| VL CDR1 | 21 | KSSQSLLYSNGKTYLN |
| | 22 | KYYASAAKLYRNIKLN |
| | 23 | KYYASLLATYRNIKLN |
| | 24 | KYYASLLATTWAKGNN |
| VL CDR2 | 27 | LVQSLDS |
| | 28 | LVSKLTA |
| | 29 | LVSKLDS |
| | 30 | LVQSDSK |
| VL CDR3 | 33 | VQGTHNYGH |
| | 34 | VGGTHFPRT |
| | 35 | VGTAIFPRT |
| | 36 | VGGTHFITA |
| | 38 | VQGTHFPRT |

In some embodiments of the first aspect of the disclosure, the soluble CD14 subtype capture antibody comprises VH CDR1, VH CDR2 and VH CDR3, as well as VL CDR1, VL CDR2 and VL CDR3, which are any one of the following 1) to 62):
1) VH CDR1 composed of the amino acid sequence of SEQ ID NO: 1, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 34;
2) VH CDR1 composed of the amino acid sequence of SEQ ID NO: 1, VH CDR2 composed of the amino acid sequence of SEQ ID NO: 11, VH CDR3 composed of the amino acid sequence of SEQ ID NO: 17, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 41;
3) VH CDR1 composed of the amino acid sequence of SEQ ID NO: 1, VH CDR2 composed of the amino acid sequence of SEQ ID NO: 12, VH CDR3 composed of the amino acid sequence of SEQ ID NO: 17, VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 33;
4) VH CDR1 composed of the amino acid sequence of SEQ ID NO: 1, VH CDR2 composed of the amino acid sequence of SEQ ID NO: 12, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 32, and VL CDR3 composed of the amino acid sequence of SEQ ID NO: 39;
5) VH CDR1 composed of the amino acid sequence of SEQ ID NO: 1, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 40;
6) VH CDR1 composed of the amino acid sequence of SEQ ID NO: 2, VH CDR2 composed of the amino acid sequence of SEQ ID NO: 7, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 35;
7) VH CDR1 composed of the amino acid sequence of SEQ ID NO: 2, VH CDR2 composed of the amino acid sequence of SEQ ID NO: 7, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 31, and VL CDR3 composed of the amino acid sequence of SEQ ID NO: 38;
8) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 14, VL CDR1 composed of the amino acid sequence of SEQ ID NO: 24, VL CDR2 composed of the amino acid sequence of SEQ ID NO: 30, and VL CDR3 composed of the amino acid sequence of SEQ ID NO: 38;
9) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 23, VL CDR2 composed of the amino acid sequence of SEQ ID NO: 29, and VL CDR3 composed of the amino acid sequence of SEQ ID NO: 40;
10) VH CDR1 composed of the amino acid sequence of SEQ ID NO: 2, VH CDR2 composed of the amino acid sequence of SEQ ID NO: 8, VH CDR3 composed of the amino acid sequence of SEQ ID NO: 17, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 38;
11) VH CDR1 composed of the amino acid sequence of SEQ ID NO: 2, VH CDR2 composed of the amino acid sequence of SEQ ID NO: 9, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 27, and VL CDR3 composed of the amino acid sequence of SEQ ID NO: 38;
12) VH CDR1 composed of the amino acid sequence of SEQ ID NO: 2, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 24, VL CDR2 composed of the amino acid sequence of SEQ ID NO: 29, and VL CDR3 composed of the amino acid sequence of SEQ ID NO: 35;
13) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 26, VL CDR2 composed of the amino acid sequence of SEQ ID NO: 29, and VL CDR3 composed of the amino acid sequence of SEQ ID NO: 36;
14) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 26, VL CDR2 composed of the amino acid sequence of SEQ ID NO: 32, and VL CDR3 composed of the amino acid sequence of SEQ ID NO: 34;
15) VH CDR1 composed of the amino acid sequence of SEQ ID NO: 2, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 26, VL CDR2 composed of the amino acid sequence of SEQ ID NO: 27, and VL CDR3 composed of the amino acid sequence of SEQ ID NO: 41;
16) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 37;
17) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 29, and VL CDR3 composed of the amino acid sequence of SEQ ID NO: 35;
18) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 40;
19) VH CDR1 composed of the amino acid sequence of SEQ ID NO: 2, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 30, and VL CDR3 composed of the amino acid sequence of SEQ ID NO: 34;
20) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 23, VL CDR2 composed of the amino acid sequence of SEQ ID NO: 32, and VL CDR3 composed of the amino acid sequence of SEQ ID NO: 34;
21) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 27, and VL CDR3 composed of the amino acid sequence of SEQ ID NO: 34;
22) VH CDR1 composed of the amino acid sequence of SEQ ID NO: 2, VH CDR2 composed of the amino acid sequence of SEQ ID NO: 13, VH CDR3 composed of the amino acid sequence of SEQ ID NO: 17, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 36;
23) VH CDR1 composed of the amino acid sequence of SEQ ID NO: 3, VH CDR2 composed of the amino acid sequence of SEQ ID NO: 7, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 41;
24) VH CDR1 composed of the amino acid sequence of SEQ ID NO: 3, VH CDR2 composed of the amino acid sequence of SEQ ID NO: 7, VH CDR3 composed of the amino acid sequence of SEQ ID NO: 19, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 34;
25) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 28, and VL CDR3 composed of the amino acid sequence of SEQ ID NO: 37;
26) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 22, VL CDR2 composed of the amino acid sequence of SEQ ID NO: 31, and VL CDR3 composed of the amino acid sequence of SEQ ID NO: 37;
27) VH CDR1 composed of the amino acid sequence of SEQ ID NO: 3, VH CDR2 composed of the amino acid sequence of SEQ ID NO: 8, VH CDR3 composed of the amino acid sequence of SEQ ID NO: 19, VL CDR1 composed of the amino acid sequence of SEQ ID NO: 25, VL CDR2 composed of the amino acid sequence of SEQ ID NO: 27, and VL CDR3 composed of the amino acid sequence of SEQ ID NO: 37;
28) VH CDR1 composed of the amino acid sequence of SEQ ID NO: 3, VH CDR2 composed of the amino acid sequence of SEQ ID NO: 9, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 29, and VL CDR3 composed of the amino acid sequence of SEQ ID NO: 36;
29) VH CDR1 composed of the amino acid sequence of SEQ ID NO: 3, VH CDR2 composed of the amino acid sequence of SEQ ID NO: 9, VH CDR3 composed of the amino acid sequence of SEQ ID NO: 16, VL CDR1 composed of the amino acid sequence of SEQ ID NO: 21, VL CDR2 composed of the amino acid sequence of SEQ ID NO: 29, and VL CDR3 composed of the amino acid sequence of SEQ ID NO: 38;
30) VH CDR1 composed of the amino acid sequence of SEQ ID NO: 3, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 41;
31) VH CDR1 composed of the amino acid sequence of SEQ ID NO: 3, VH CDR2 composed of the amino acid sequence of SEQ ID NO: 12, VH CDR3 composed of the amino acid sequence of SEQ ID NO: 15, VL CDR1 composed of the amino acid sequence of SEQ ID NO: 24, VL CDR2 composed of the amino acid sequence of SEQ ID NO: 32, and VL CDR3 composed of the amino acid sequence of SEQ ID NO: 39;
32) VH CDR1 composed of the amino acid sequence of SEQ ID NO: 4, VH CDR2 composed of the amino acid sequence of SEQ ID NO: 7, VH CDR3 composed of the amino acid sequence of SEQ ID NO: 15, VL CDR1 composed of the amino acid sequence of SEQ ID NO: 25, VL CDR2 composed of the amino acid sequence of SEQ ID NO: 28, and VL CDR3 composed of the amino acid sequence of SEQ ID NO: 37;
33) VH CDR1 composed of the amino acid sequence of SEQ ID NO: 4, VH CDR2 composed of the amino acid sequence of SEQ ID NO: 7, VH CDR3 composed of the amino acid sequence of SEQ ID NO: 18, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 33;
34) VH CDR1 composed of the amino acid sequence of SEQ ID NO: 4, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 40;
35) VH CDR1 composed of the amino acid sequence of SEQ ID NO: 4, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 37;
36) VH CDR1 composed of the amino acid sequence of SEQ ID NO: 4, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 33;
37) VH CDR1 composed of the amino acid sequence of SEQ ID NO: 4, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 24, VL CDR2 composed of the amino acid sequence of SEQ ID NO: 27, and VL CDR3 composed of the amino acid sequence of SEQ ID NO: 34;
38) VH CDR1 composed of the amino acid sequence of SEQ ID NO: 4, VH CDR2 composed of the amino acid sequence of SEQ ID NO: 13, VH CDR3 composed of the amino acid sequence of SEQ ID NO: 16, VL CDR1 composed of the amino acid sequence of SEQ ID NO: 22, VL CDR2 composed of the amino acid sequence of SEQ ID NO: 32, and VL CDR3 composed of the amino acid sequence of SEQ ID NO: 40;
39) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 40;
40) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 34;
41) VH CDR1 composed of the amino acid sequence of SEQ ID NO: 5, VH CDR2 composed of the amino acid sequence of SEQ ID NO: 7, VH CDR3 composed of the amino acid sequence of SEQ ID NO: 20, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
42) VH CDR1 composed of the amino acid sequence of SEQ ID NO: 5, VH CDR2 composed of the amino acid sequence of SEQ ID NO: 8, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
43) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
44) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 15, VL CDR1 composed of the amino acid sequence of SEQ ID NO: 21, VL CDR2 composed of the amino acid sequence of SEQ ID NO: 28, and VL CDR3 composed of the amino acid sequence of SEQ ID NO: 38;
45) VH CDR1 composed of the amino acid sequence of SEQ ID NO: 5, VH CDR2 composed of the amino acid sequence of SEQ ID NO: 10, VH CDR3 composed of the amino acid sequence of SEQ ID NO: 19, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 40;
46) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 21, VL CDR2 composed of the amino acid sequence of SEQ ID NO: 31, and VL CDR3 composed of the amino acid sequence of SEQ ID NO: 33;
47) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 31, and VL CDR3 composed of the amino acid sequence of SEQ ID NO: 36;
48) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 17, VL CDR1 composed of the amino acid sequence of SEQ ID NO: 23, VL CDR2 composed of the amino acid sequence of SEQ ID NO: 28, and VL CDR3 composed of the amino acid sequence of SEQ ID NO: 39;
49) VH CDR1 composed of the amino acid sequence of SEQ ID NO: 5, VH CDR2 composed of the amino acid sequence of SEQ ID NO: 11, VH CDR3 composed of the amino acid sequence of SEQ ID NO: 16, VL CDR1 composed of the amino acid sequence of SEQ ID NO: 25, VL CDR2 composed of the amino acid sequence of SEQ ID NO: 29, and VL CDR3 composed of the amino acid sequence of SEQ ID NO: 38;
50) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 17, VL CDR1 composed of the amino acid sequence of SEQ ID NO: 21, VL CDR2 composed of the amino acid sequence of SEQ ID NO: 27, and VL CDR3 composed of the amino acid sequence of SEQ ID NO: 37;
51) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 19, VL CDR1 composed of the amino acid sequence of SEQ ID NO: 26, VL CDR2 composed of the amino acid sequence of SEQ ID NO: 31, and VL CDR3 composed of the amino acid sequence of SEQ ID NO: 39;
52) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 16, VL CDR1 composed of the amino acid sequence of SEQ ID NO: 23, VL CDR2 composed of the amino acid sequence of SEQ ID NO: 27, and VL CDR3 composed of the amino acid sequence of SEQ ID NO: 34;
53) VH CDR1 composed of the amino acid sequence of SEQ ID NO: 5, VH CDR2 composed of the amino acid sequence of SEQ ID NO: 12, VH CDR3 composed of the amino acid sequence of SEQ ID NO: 19, VL CDR1 composed of the amino acid sequence of SEQ ID NO: 24, VL CDR2 composed of the amino acid sequence of SEQ ID NO: 31, and VL CDR3 composed of the amino acid sequence of SEQ ID NO: 38;
54) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 26, VL CDR2 composed of the amino acid sequence of SEQ ID NO: 30, and VL CDR3 composed of the amino acid sequence of SEQ ID NO: 39;
55) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 15, VL CDR1 composed of the amino acid sequence of SEQ ID NO: 26, VL CDR2 composed of the amino acid sequence of SEQ ID NO: 28, and VL CDR3 composed of the amino acid sequence of SEQ ID NO: 33;
56) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 18, VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
57) VH CDR1 composed of the amino acid sequence of SEQ ID NO: 5, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
58) VH CDR1 composed of the amino acid sequence of SEQ ID NO: 6, VH CDR2 composed of the amino acid sequence of SEQ ID NO: 7, VH CDR3 composed of the amino acid sequence of SEQ ID NO: 18, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
59) VH CDR1 composed of the amino acid sequence of SEQ ID NO: 6, VH CDR2 composed of the amino acid sequence of SEQ ID NO: 10, VH CDR3 composed of the amino acid sequence of SEQ ID NO: 14, VL CDR1 composed of the amino acid sequence of SEQ ID NO: 25, VL CDR2 composed of the amino acid sequence of SEQ ID NO: 32, and VL CDR3 composed of the amino acid sequence of SEQ ID NO: 39;
60) VH CDR1 composed of the amino acid sequence of SEQ ID NO: 6, VH CDR2 composed of the amino acid sequence of SEQ ID NO: 11, VH CDR3 composed of the amino acid sequence of SEQ ID NO: 18, VL CDR1 composed of the amino acid sequence of SEQ ID NO: 22, VL CDR2 composed of the amino acid sequence of SEQ ID NO: 29, and VL CDR3 composed of the amino acid sequence of SEQ ID NO: 39;
61) VH CDR1 composed of the amino acid sequence of SEQ ID NO: 6, VH CDR2 composed of the amino acid sequence of SEQ ID NO: 11, VH CDR3 composed of the amino acid sequence of SEQ ID NO: 14, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 39; or
62) VH CDR1 composed of the amino acid sequence of SEQ ID NO: 6, VH CDR2 composed of the amino acid sequence of SEQ ID NO: 13, VH CDR3 composed of the amino acid sequence of SEQ ID NO: 20, VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 38.

In some embodiments of the first aspect of the disclosure, the soluble CD14 subtype capture antibody comprises:
(a) VH comprising VH CDR1 composed of the amino acid sequence of SEQ ID NO: 1, VH CDR2 composed of the amino acid sequence of SEQ ID NO: 12, and VH CDR3 composed of the amino acid sequence of SEQ ID NO: 17, and VL comprising VL CDR1 composed of the amino acid sequence of SEQ ID NO: 21, VL CDR2 composed of the amino acid sequence of SEQ ID NO: 32, and VL CDR3 composed of the amino acid sequence of SEQ ID NO: 33;
(b) VH comprising VH CDR1 composed of the amino acid sequence of SEQ ID NO: 2, VH CDR2 composed of the amino acid sequence of SEQ ID NO: 12, and VH CDR3 composed of the amino acid sequence of SEQ ID NO: 20, and VL comprising VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 37;
(c) VH comprising VH CDR1 composed of the amino acid sequence of SEQ ID NO: 3, VH CDR2 composed of the amino acid sequence of SEQ ID NO: 9, and VH CDR3 composed of the amino acid sequence of SEQ ID NO: 16, and VL comprising VL CDR1 composed of the amino acid sequence of SEQ ID NO: 21, VL CDR2 composed of the amino acid sequence of SEQ ID NO: 29, and VL CDR3 composed of the amino acid sequence of SEQ ID NO: 38;
(d) VH comprising VH CDR1 composed of the amino acid sequence of SEQ ID NO: 5, VH CDR2 composed of the amino acid sequence of SEQ ID NO: 13, and VH CDR3 composed of the amino acid sequence of SEQ ID NO: 18, and VL comprising VL CDR1 composed of the amino acid sequence of SEQ ID NO: 21, VL CDR2 composed of the amino acid sequence of SEQ ID NO: 30, and VL CDR3 composed of the amino acid sequence of SEQ ID NO: 39;
(e) VH comprising VH CDR1 composed of the amino acid sequence of SEQ ID NO: 4, VH CDR2 composed of the amino acid sequence of SEQ ID NO: 12, and VH CDR3 composed of the amino acid sequence of SEQ ID NO: 16, and VL comprising VL CDR1 composed of the amino acid sequence of SEQ ID NO: 23, VL CDR2 composed of the amino acid sequence of SEQ ID NO: 30, and VL CDR3 composed of the amino acid sequence of SEQ ID NO: 33; or
(f) VH comprising VH CDR1 composed of the amino acid sequence of SEQ ID NO: 6, VH CDR2 composed of the amino acid sequence of SEQ ID NO: 13, and VH CDR3 composed of the amino acid sequence of SEQ ID NO: 20, and VL comprising VL CDR1 composed of the amino acid sequence of SEQ ID NO: 22, VL CDR2 composed of the amino acid sequence of SEQ ID NO: 30, and VL CDR3 composed of the amino acid sequence of SEQ ID NO: 38.

In some embodiments of the first aspect of the disclosure, a Presepsin capture antibody may be derived from an anti-soluble CD14 subtype monoclonal antibody or an antigen-binding antibody fragment thereof secreted and produced by a hybridoma cell strain with the deposit number of CGMCC NO.18536, and a Presepsin detection antibody may be derived from an anti-soluble CD14 subtype polyclonal antibody secreted and produced by a hybridoma cell strain with the deposit number of CGMCC NO.18536.

In some embodiments of the first aspect of the disclosure, a solid phase carrier may be selected from a magnetic microsphere, a chip, a test paper, etc., preferably a magnetic microsphere, and more preferably a superparamagnetic bead.

In some embodiments of the first aspect of the disclosure, a PCT detection antibody and a Presepsin detection antibody are labeled with a signal label, which may be, for example, a chemiluminescent label (such as an alkaline phosphatase, luminol, isoluminol, an acridinium ester, and a horseradish peroxidase), an electrochemiluminescent label (e.g., ruthenium terpyridine), a quantum dot (such as a gold quantum dot, a CdSe quantum dot, and a ZnCdSe quantum dot), a fluorescent microsphere, or a combination thereof.

In some embodiments of the first aspect of the disclosure, a capture antibody mixture may further include a capture antibody specific for other markers, and accordingly, a detection antibody mixture comprises a detection antibody specific for the other markers, where the other markers may be selected from at least one of C-reactive protein (CRP), interleukin-6 (IL-6), tumor necrosis factor (TNF-α), serum athermoprotein A (SAA), heparin-binding protein (HBP), soluble urokinase-type plasminogen activator receptor (suPAR), interleukin-1β (IL-1β), interleukins such as IL-2, IL-4, IL-8, IL-10, IL-12, IL-13 and IL-18, tissue inhibitor of metalloproteinase-1 (TIMP-1), soluble triggering receptor expressed on myeloid cells-1 (sTREM-1), matrix metalloproteinase 9 (MMP-9), pro-adrenomedullin (Pro-ADM), Toll-like receptor (TLR2), α2-macroglobulin (A2M), troponin I (TnI), C-C chemokine ligand 2 (CCL2), CD163, osteopontin (OPN), D-Dimer, lipopolysaccharide binding protein (LBP), (1,3)-β-D-glucan (BG), coagulation factor, angiopoietin-1 (Ang-1), angiopoietin-2 (Ang-2), vascular endothelial growth factor, γ-interferon human, immunoglobulin E, human immunoglobulin A, human immunoglobulin M, human immunoglobulin G, and human urinary microalbumin. Preferably, the other markers may be selected from at least one of C-reactive protein (CRP), interleukin-6 (IL-6), tumor necrosis factor (TNF-α), serum athermoprotein A (SAA), and heparin-binding protein (HBP).

In some embodiments of the first aspect of the disclosure, a PCT capture antibody may also be replaced with a capture antibody specific for other markers, and accordingly, a detection antibody mixture comprises a detection antibody specific for the other markers instead of a PCT detection antibody, where the other markers may be, for example, one of the markers listed above.

The second aspect of the disclosure relates to a method for combined detection of PCT and Presepsin in a sample to be tested, including the following steps:
mixing a sample to be tested, a procalcitonin capture antibody coated on a solid phase, and a soluble CD14 subtype capture antibody coated on a solid phase, so that the procalcitonin capture antibody coated on the solid phase binds to the procalcitonin in the sample to be tested, and the soluble CD14 subtype capture antibody coated on the solid phase binds to the soluble CD14 subtype in the sample to be tested;
adding a labeled procalcitonin detection antibody and a labeled soluble CD14 subtype detection antibody to the mixture of the sample to be tested, the procalcitonin capture antibody, and the soluble CD14 subtype capture antibody, so that the labeled procalcitonin detection antibody binds to the procalcitonin bound on the procalcitonin capture antibody to form a sandwich complex and the labeled soluble CD14 subtype detection antibody binds to the soluble CD14 subtype bound on the soluble CD14 subtype capture antibody to form a sandwich complex;
washing the sandwich complexes to remove unbound substances;
adding a detection substrate to the sandwich complexes to detect the combined concentration level of the procalcitonin and the soluble CD14 subtype in the sample to be tested;
wherein the procalcitonin detection antibody and the soluble CD14 subtype detection antibody has a same label, wherein the label is an enzyme, and the detection substrate is a substrate for the enzyme.

In some embodiments of the second aspect of the disclosure, the method specifically includes:
mixing a sample to be tested, a procalcitonin capture antibody coated on a solid phase, and a soluble CD14 subtype capture antibody coated on a solid phase, so that the procalcitonin capture antibody coated on the solid phase binds to the procalcitonin in the sample to be tested, and the soluble CD14 subtype capture antibody coated on the solid phase binds to the soluble CD14 subtype in the sample to be tested;
washing the mixture of the sample to be tested, the procalcitonin capture antibody and the soluble CD14 subtype capture antibody to remove unbound substances;
adding a labeled procalcitonin detection antibody and a labeled soluble CD14 subtype detection antibody to the washed mixture, so that the labeled procalcitonin detection antibody binds to the procalcitonin bound on the procalcitonin capture antibody to form a sandwich complex and the labeled soluble CD14 subtype detection antibody binds to the soluble CD14 subtype bound on the soluble CD14 subtype capture antibody to form a sandwich complex;
washing the sandwich complex to remove unbound substances;
adding a chemiluminescent substrate to the washed sandwich complexes to detect the number of photons generated by the reaction, thereby obtaining a combined signal value of the procalcitonin and the soluble CD14 subtype in the sample to be tested, where the combined signal value represents the combined concentration level of the procalcitonin and the soluble CD14 subtype in the sample to be tested, which is directly proportional to the combined concentration value of the procalcitonin and the soluble CD14 subtype in the sample to be tested;
wherein the procalcitonin detection antibody and the soluble CD14 subtype detection antibody has a same label, wherein the label is an enzyme, and the chemiluminescent substrate is a substrate for the enzyme.

In some embodiments of the second aspect of the disclosure, the procalcitonin capture antibody and the soluble CD14 subtype capture antibody are separately coated on different solid phases. Alternatively, the procalcitonin capture antibody and the soluble CD14 subtype capture antibody may also be co-coated on the same solid phase.

In some embodiments of the second aspect of the disclosure, a PCT capture antibody and a Presepsin capture antibody are added to a sample to be tested together or separately.

In some embodiments of the second aspect of the disclosure, a PCT detection antibody and a Presepsin detection antibody are added to a sample to be tested together or separately.

In some embodiments of the second aspect of the disclosure, the addition ratio of a PCT capture antibody to a Presepsin capture antibody is determined according to the principle of maximizing the diagnostic efficacy for diagnosing sepsis, that is, the mixing ratio is selected according to the principle of maximizing the AUC of the ROC curve. The ratio of a PCT capture antibody to a Presepsin capture antibody which are added to the sample to be tested may be in the range of about 5: 1 to about 1: 5, preferably in the range of about 3: 1 to about 1:5, more preferably in the range of about 3: 1 to 1: 3, in particular about 3: 1 or 1: 1 or 1: 3, and more particularly preferably about 1:3.

In some embodiments of the second aspect of the disclosure, the addition ratio of a PCT detection antibody to a Presepsin detection antibody is determined according to the principle of maximizing the diagnostic efficacy for diagnosing sepsis, that is, the mixing ratio is selected according to the principle of maximizing the AUC of the ROC curve. The amount ratio of a PCT detection antibody to a Presepsin detection antibody which are added to the sample to be tested may be in the range of about 5: 1 to about 1: 5, preferably in the range of about 3: 1 to about 1:5, more preferably in the range of about 3: 1 to 1: 3, in particular about 3: 1 or 1: 1 or 1: 3, and more particularly preferably about 1:3.

In some embodiments of the second aspect of the disclosure, the addition ratio of a PCT capture antibody to a Presepsin capture antibody may be equal to the addition ratio of a PCT detection antibody to a Presepsin detection antibody, and the addition ratios are preferably 1: 3.

In some embodiments of the second aspect of the disclosure, a sample to be tested may be blood and a blood component such as serum or plasma, preferably a plasma sample.

For other features of the second aspect of the disclosure, such as a PCT capture antibody/detection antibody, a Presepsin capture antibody/detection antibody, a label, and a solid phase, reference may be made to the related description in the first aspect of the disclosure, and details are not described herein again.

The third aspect of the disclosure relates to the use of the soluble CD14 subtype antibody and the procalcitonin antibody in the preparation of an analysis reagent for identifying whether a patient suffers from sepsis, where a sample to be tested from the patient, a procalcitonin capture antibody coated on the solid phase and a soluble CD14 subtype capture antibody coated on the solid phase are mixed, and then a labeled procalcitonin detection antibody and a labeled soluble CD14 subtype detection antibody are added to detect a combined concentration level of the procalcitonin and the soluble CD14 subtype in the sample to be tested, and an increase in the combined concentration level relative to the reference value is associated with an increase in possibility that the patient suffers from sepsis;
wherein the procalcitonin detection antibody and the soluble CD14 subtype detection antibody are labeled with a same label.

In some embodiments of the third aspect of the disclosure, a chemiluminescent substrate is used to detect the number of photons generated by the reaction of a sample to be tested from a patient, a capture antibody and a detection antibody, thereby obtaining a combined signal value of the procalcitonin and the soluble CD14 subtype in the sample to be tested, where the combined signal value represents the combined concentration value of the procalcitonin and the soluble CD14 subtype in the sample to be tested. The combined signal value of PCT and Presepsin in a sample to be tested may be compared with a threshold value to obtain a combined detection factor, for example, combined detection factor = combined signal value of a sample to be tested/threshold value. The combined detection factor is then compared with a reference value to evaluate whether a patient suffers from sepsis.

In some embodiments of the third aspect of the disclosure, the increase in the combined signal value relative to a reference value is positively correlated with an increase in possibility that a patient suffers from sepsis.

In some embodiments of the third aspect of the disclosure, a sample to be tested is detected with a combined detection kit according to the disclosure, and an ROC curve is established. According to the ROC curve, a threshold value is determined, and the combined signal value is compared with the threshold value. When a combined detection factor is ≥ 1, a patient is at high risk of suffering from sepsis; when the combined detection factor is < 1, the patient is at low risk of suffering from sepsis.

In some embodiments of the third aspect of the disclosure, the combined signal value of the procalcitonin and the soluble CD14 subtype in the sample to be tested from a patient is detected within 72 hours after the patient is suspected of suffering from sepsis.

A fourth aspect of the disclosure relates to use of a soluble CD14 subtype antibody and a procalcitonin antibody in the preparation of an analysis reagent for evaluating the prognosis of a suspected sepsis patient, where a sample to be tested from the patient, a procalcitonin capture antibody coated on a solid phase and a soluble CD14 subtype capture antibody coated on a solid phase are mixed, and then a labeled procalcitonin detection antibody and a labeled soluble CD 14 subtype detection antibody are added to detect a combined concentration level of the procalcitonin and the soluble CD14 subtype in the sample to be tested, and an increase in the combined concentration level relative to a reference value is associated with an increase in risk of death in the suspected sepsis patient;
wherein the procalcitonin detection antibody and the soluble CD14 subtype detection antibody are labeled with a same label.

In some embodiments of the fourth aspect of the disclosure, a chemiluminescent substrate is used to detect the number of photons generated by the reaction of a sample to be tested from a patient, a capture antibody and a detection antibody, thereby obtaining a combined signal value of the procalcitonin and the soluble CD14 subtype in the sample to be tested, where the combined signal value represents the combined concentration value of the procalcitonin and the soluble CD14 subtype in the sample to be tested. The combined signal value of PCT and Presepsin in a sample to be tested may be compared with a threshold value to obtain a combined detection factor, for example, combined detection factor = combined signal value of a sample to be tested/threshold value. The combined detection factor is then compared with a reference value to evaluate the risk of death in a suspected sepsis patient.

In some embodiments of the fourth aspect of the disclosure, the increase in the combined signal value relative to a reference value is positively correlated with an increase in risk of death in a suspected sepsis patient.

In some embodiments of the fourth aspect of the disclosure, the combined signal value of the procalcitonin and the soluble CD14 subtype in the sample to be tested from a patient is detected within 72 hours after the patient is suspected of suffering from sepsis.

### Example 1: Preparation of a kit (a combined detection kit) for combined detection of PCT and Presepsin in a sample to be tested

The combined detection kit of the disclosure was prepared by the following method: a PCT capture antibody and a Presepsin capture antibody were mixed at a certain ratio, and then the mixture was coated on the surface of a solid phase carrier; and next, a PCT detection antibody and a Presepsin detection antibody were mixed at a certain ratio, and then the mixture was labeled with a signal label.

First, a PCT capture antibody and a Presepsin capture antibody were mixed at a concentration ratio of about 5: 1 to about 1: 5, respectively, and the mixture was coated on the surface of a solid phase carrier, where the solid phase carrier was preferably a superparamagnetic bead. A PCT detection antibody and a Presepsin detection antibody were also mixed at a ratio of about 5: 1 to about 1:5, and the mixture was labeled with a signal label, where the signal label is preferably an alkaline phosphatase. In this example, the ratio of the PCT capture antibody to the Presepsin capture antibody was the same as the ratio of the PCT detection antibody to the Presepsin detection antibody. Then, the coated capture antibody mixture and the labeled detection antibody mixture were diluted with a buffer containing about 50 mM of MES (2-morpholinoethanesulfonic acid) and about 0.5 M of NaCl, with pH of about 6.0. Upon dilution, the concentration of the coated capture antibody mixture was in the range of about 1 µg/mL to about 10 µg/mL, preferably about 2 µg/mL, and the concentration of the labeled detection antibody mixture was in the range of about 0.5 µg/mL to about 5 µg/mL, preferably about 1 µg/mL.

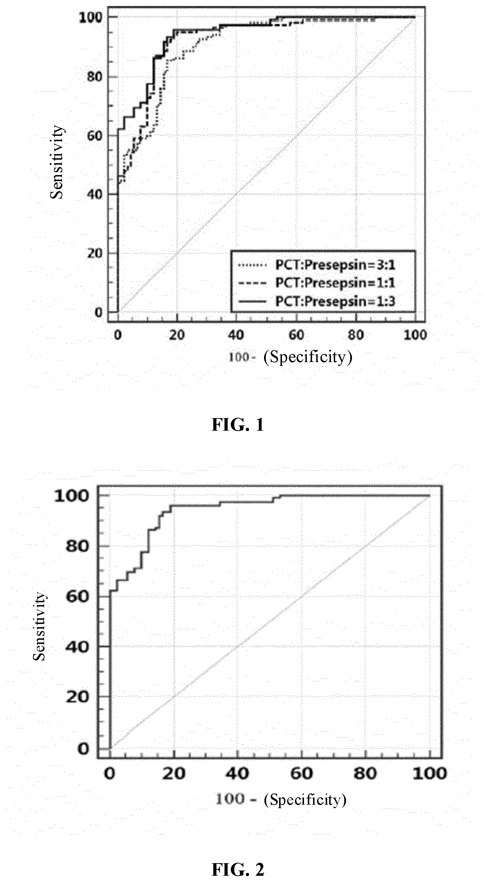

Samples with known clinical diagnostic results were selected, including > 50 negative samples for sepsis and > 50 positive samples for sepsis. The kit prepared by the method described above was selected to detect a sample to be tested, and an ROC curve was established to calculate the AUC area, as shown in Table 3 and FIG. 1.

**Table 3**

| Concentration ratio of PCT capture antibody to Presepsin capture antibody | Concentration ratio of PCT detection antibody to Presepsin detection antibody | AUC area |
|---|---|---|
| 1: 0 | 1: 0 | 0.841 |
| 5: 1 | 5: 1 | 0.906 |
| 3: 1 | 3: 1 | 0.912 |
| 1: 1 | 1: 1 | 0.926 |
| 1: 3 | 1: 3 | 0.945 |
| 1: 5 | 1: 5 | 0.929 |
| 0: 1 | 0: 1 | 0.913 |

It can be seen from Table 3 and FIG. 1 that within a suitable concentration ratio of the PCT capture antibody to the Presepsin capture antibody or the concentration ratio of the PCT detection antibody to the Presepsin detection antibody, there is a larger the corresponding AUC area, and the diagnostic efficacy for diagnosing sepsis will be greater. When the concentration ratio of the PCT capture antibody to the Presepsin capture antibody or the concentration ratio of the PCT detection antibody to the Presepsin detection antibody reaches a certain value, the AUC area no longer increases. In the embodiments of the disclosure, when the mixing ratio of the PCT capture antibody to the Presepsin capture antibody and the mixing ratio of the PCT detection antibody to the Presepsin detection antibody are both about 1:3, the diagnostic efficacy for diagnosing sepsis is relatively maximum.

In subsequent experiments, the mixing ratio of the PCT capture antibody to the Presepsin capture antibody was selected to be about 1: 3, and similarly, the mixing ratio of the PCT detection antibody to the Presepsin detection antibody was selected to be about 1: 3.

### Example 2: Method for combined detection of PCT and Presepsin in a sample to be tested

A capture antibody mixture coated on a solid phase carrier and a labeled detection antibody mixture in the combined detection kit according to the embodiments of the disclosure, for example, the combined detection kit prepared according to Example 1 were mixed with a sample to be tested, where the PCT biomarker in the sample to be tested was bound with the PCT capture antibody and the PCT detection antibody to form a sandwich structure; similarly, the Presepsin biomarker in the sample to be tested was bound with the Presepsin capture antibody and the Presepsin detection antibody to form a sandwich structure; an unbound detection antibody was removed by washing; the signal generated by a signal label labeled with a detection antibody was detected by an instrument for example, CL-2000i, CL-900 i, CL-6000 i or CL-1000 i chemiluminescent analyzer from Shenzhen Mindray Bio-medical Electronics Co., Ltd., thereby obtaining a combined signal value of PCT and Presepsin in the sample to be tested, where the intensity of the combined signal value is directly proportional to the concentration of Presepsin and PCT in the sample to be tested. 215 samples with known clinical diagnostic results were selected, including 95 negative samples for sepsis and 120 positive samples for sepsis. With the help of a detection device, such as the CL-2000i analyzer from Shenzhen Mindray Bio-medical Electronics Co., Ltd., the above-mentioned 215 samples were detected by the method of this example, and an ROC curve was established, as shown in FIG. 2. In this example, according to the ROC curve as shown in FIG. 2, a threshold value was determined to be 200,000, and the sensitivity and specificity corresponding to the threshold value were: 0.86 and 0.87, respectively.

### Example 3: Use of a Presepsin antibody and a PCT antibody in the preparation of an analysis reagent for identifying whether a patient suffers from sepsis

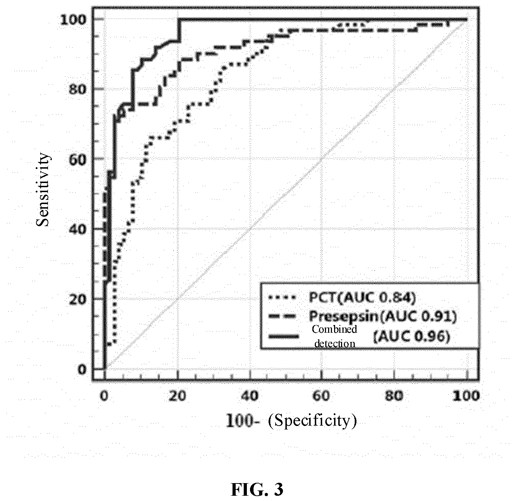

By using the method of the embodiments of the disclosure, the combined detection kit according to the embodiments of the disclosure was used to detect plasma samples from 141 suspected sepsis patients within 72 hours after admission to hospital, thereby obtaining a combined signal value; and in addition, the concentration values of PCT and Presepsin in the plasma samples from 141 suspected sepsis patients were detected, respectively. According to the detection results, ROC curves corresponding to detection of PCT alone, detection of Presepsin alone, and combined detection of PCT and Presepsin were established, respectively, as shown in FIG. 3. According to the AUC area of the ROC curve, the AUC areas of the ROC curves for detection of PCT alone, detection of Presepsin alone, and combined detection of PCT and Presepsin were 0.84, 0.91 and 0.96, respectively. It can be seen therefrom that the diagnostic efficacy of combined detection of PCT and Presepsin is higher.

The combined detection kit according to the embodiments of the disclosure was used to detect 200 samples, including 60 healthy people, 52 people with local infection, 51 patients with sepsis, and 37 people with septic shock. The diagnostic criteria are based on Sepsis 3.0 (Singer M, Deutschman CS, Seymour CW, et al. The Third international consensus definitions for sepsis and septic shock (Sepsis-3) [J]), and the detection results of different groups (see Table 4) show that with the development of infection, the value of the combined detection factor is increasing.

**Table 4**

| Group | Number of samples | Combined detection factor | |
|---|---|---|---|
| | | 2.5th percentile | 97.5th percentile |
| Healthy people | 60 | 0.02 | 0.05 |
| People with local infection | 52 | 0.17 | 0.65 |
| Patient with sepsis | 51 | 0.93 | 11.96 |
| People with septic shock | 37 | 10.11 | 151.85 |

### Example 4: Use of a Presepsin antibody and a PCT antibody in the preparation of an analysis reagent for evaluating the prognosis of a suspected sepsis patient

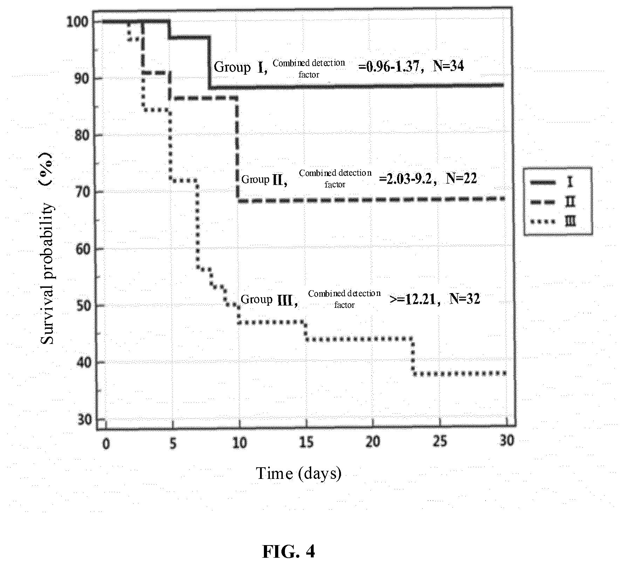

By using the method of the embodiments of the disclosure, the combined detection kit according to the embodiments of the disclosure was used to detect plasma samples from 88 suspected sepsis patients and the prognosis of the patients within 30 days was tracked. The relationship graph of survival probability curve within 30 days after the suspected sepsis patients were admitted to hospital was obtained by using a Kaplan-Meier statistical analysis method, as shown in FIG. 4, where N represented the number of patients. For different groups, the survival probability of patients was significantly different, and for group III, when the combined detection factor was ≥ 12.21, the survival probability of patients was less than 40% (p < 0.01).

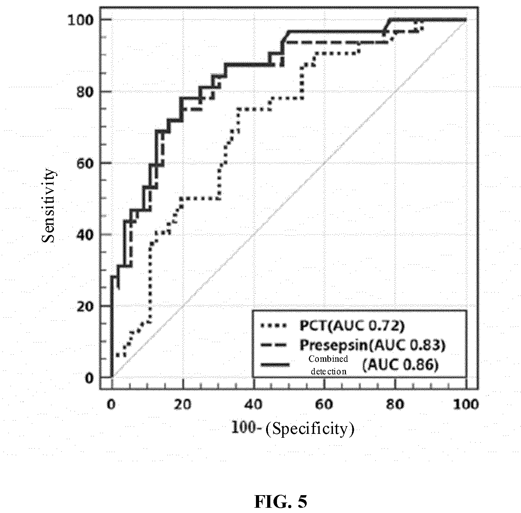

In addition, taking the above-mentioned 88 suspected sepsis patients as the object, an ROC curve was used to compare the concentration values of combined detection, detection of PCT alone, and detection of Presepsin alone in patients within 72 hours after admission to hospital with the prognostic power of survival probability in patients within 30 days after admission to hospital. As shown in FIG. 5, the AUCs for detection of PCT alone, detection of Presepsin alone, and combined detection of PCT and Presepsin were 0.72, 0.83 and 0.86, respectively. It can be seen therefrom that the embodiments of the disclosure may more accurately predict the survival status of a suspected sepsis patient.

### Example 5: Preparation of an anti-soluble CD14 subtype antibody

### Step 1.1 Expression of a soluble CD14 subtype immunogen

The soluble CD14 subtype immunogen is expressed by using a prokaryotic expression system and a eukaryotic expression system, respectively. The corresponding process is described below in detail.

### (1) Expression of a soluble CD14 subtype immunogen by using a prokaryotic expression system

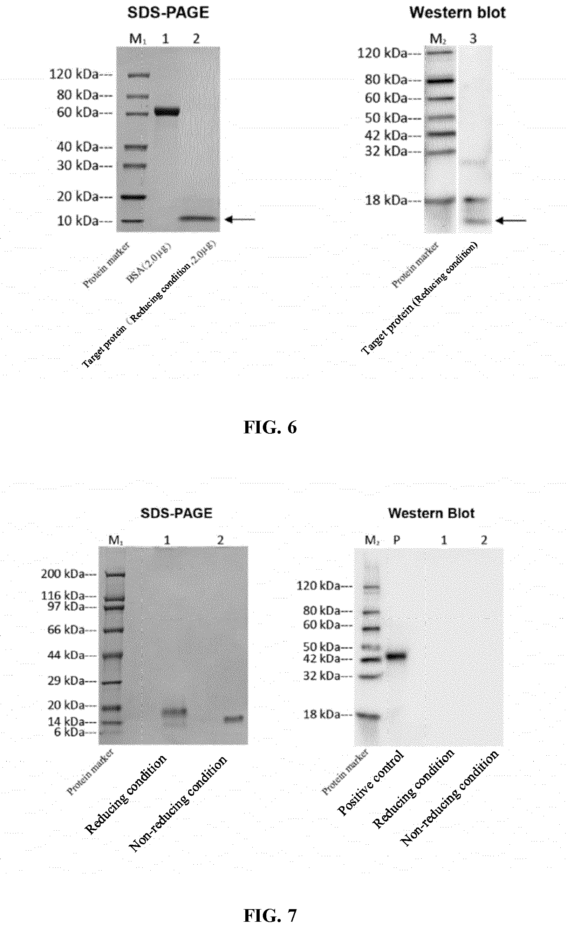

A target DNA sequence was synthesized, and then a pET30a expression vector was selected to construct a plasmid which was transformed into E. coli BL21star (DE3) to obtain a prokaryotic expression engineering strain. The recombinant plasmid was induced to be highly expressed in an E. coli expression system by an IPTG (isopropyl thiogalactoside) method. The target protein which mainly existed in an inclusion body, was purified and obtained by a Ni²⁺ NTA affinity column. The target protein had a sequence of Thr Thr Pro Glu Pro Cys Glu Leu Asp Asp Glu Asp Phe Arg Cys Val Cys Asn Phe Ser Glu Pro Gln Pro Asp Trp Ser Glu Ala Phe Gln Cys Val Ser Ala Val Glu Val Glu Ile His Ala Gly Gly Leu Asn Leu Glu Pro Phe Leu Lys Arg Val Asp Ala Asp Ala Asp Pro Arg Gln Tyr Ala (SEQ ID No. 43). As shown in FIG. 6, the concentration of the target protein was 5.42 mg/mL as determined by a Bradford method, and the purity and molecular weight thereof were determined by using SDS-PAGE and Western Blot.

### (2) Expression of a soluble CD14 subtype immunogen by using a eukaryotic expression system

A target DNA sequence was synthesized, and then a pcDNA3.4 expression vector was selected to construct a plasmid. An HEK293-6E expression system was used, and the culture conditions were as follows: the expression system was cultured in serum-free FreeStyle^{™} 293 Expression Medium (Thermo Fisher Scientific) matrix at 37°C and 5% CO2. The constructed plasmid was transfected in the 293-6E expression system by using a transient transfection method. After 6 days of culture, a cell culture supernatant was collected. The supernatant was filtered and subjected to affinity purification. The purified protein was digested by using optimized experimental conditions, and a target protein was obtained after purification. As shown in FIG. 7, the concentration of the target protein was 1.55 mg/mL as determined by a Bradford method, and the purity and molecular weight thereof were determined by using SDS-PAGE and Western Blot.

### Step 1.2 Preparation of an anti-soluble CD14 subtype polyclonal antibody

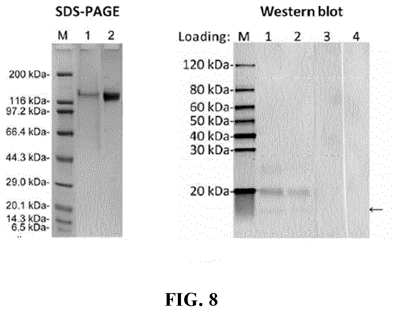

The proteins (i.e., antigens) expressed and purified by the prokaryotic system and the eukaryotic system obtained in Step 1.1 were mixed at a certain ratio, and the mixture was coupled with a carrier protein keyhole limpet hemocyanin (referred to as KLH for short), so that the resulting product was used as an immunogen to immunize New Zealand white rabbits according to the following steps: the immunogen was diluted with physiological saline, and then mixed with an adjuvant such as an incomplete Freund's adjuvant (Sigma-Aldrich) at 1: 1. The antigen and the adjuvant were thoroughly mixed to form a stable emulsion, which was injected subcutaneously under the skin around the shoulders of New Zealand white rabbits and intramuscularly in the hind thighs thereof. Each region was immunized with about 1/4 of the immunogen, and the antigen amount of each immunization was about 100 µg to 500 µg, with a total of four immunizations and an interval of each immunization of 2 weeks. After the fourth immunization, the antiserum of New Zealand white rabbits was collected and subjected to antigen affinity purification to obtain a soluble CD14 subtype polyclonal antibody. The results of SDS-PAGE and Western Blot for the anti-soluble CD14 subtype polyclonal antibody are shown in FIG. 8. In the SDS-PAGE panel on the left side of FIG. 3, lane M is a Protein marker; the first lane is a polyclonal antibody; and the second lane is a rabbit IgG. In the Western Blot panel on the right side of FIG. 8, lane M is a Protein marker; the first lane is an immunogen (100 ng) + a purified antibody (1 µg/mL) + a goat anti-rabbit IgG [IRDye800cw] (0.125 µg/mL); the second lane is an immunogen (50 ng) + a purified antibody (1 µg/mL) + a goat anti-rabbit IgG [IRDye800cw] (0.125 µg/mL); the third lane is an immunogen (100 ng) + an unimmunized serum + a goat anti-rabbit IgG [IRDye800cw] (0.125 µg/mL); and the fourth lane is an immunogen (100 ng) + a goat anti-rabbit IgG [IRDye800cw] (0.125 µg/mL).

### Step 1.3 Preparation of an anti-soluble CD14 subtype monoclonal antibody

The proteins (i.e., antigens) expressed and purified by the prokaryotic system and the eukaryotic system obtained in Step 1.1 were mixed at a certain ratio, and the mixture was coupled with a carrier protein keyhole limpet hemocyanin (referred to as KLH for short), so that the resulting product was used as an immunogen to immunize mice according to the following steps: at an initial immunization, the antigen (10 µg to 50 µg) was mixed with an adjuvant such as a complete Freund's adjuvant (Sigma-Aldrich) at 1: 1, followed by subcutaneous multi-point injection. After 3 weeks, a second immunization was performed, whose dose was the same as that at the initial immunization, and an incomplete Freund's adjuvant (Sigma-Aldrich) was added for subcutaneous injection. After another 3 weeks, a third immunization was performed, whose dose was the same as that at the initial immunization, without the addition of an adjuvant. After 3 weeks, a booster immunization was performed at doses ranging from 100 µg to 500 µg. Three days after the booster immunization, the spleen was obtained for fusion. After the third immunization, the titer of antiserum in mice was evaluated by ELISA, and the spleen cells of the animal with the best titer were selected to be fused with myeloma cells to obtain a parent clone cell strain, with the deposit number of CGMCC NO. 18536. In the scope of the disclosure, various well-known cells might be used as myeloma cells, for example, P3, NS-1, P3U1 and SP2/0 derived from mice, YB2/0 and Y3-Ag1 derived from rats, SKO-007 derived from human, and human-mouse hybrid myeloma cell SHM-D33. SP2/0 derived from mice was preferably used in the disclosure.

Then, the parent clone was screened positively and reversely by an ELISA method (an indirect ELISA method was used in both positive and reverse screening experiments).

For a positive screening experiment, the antigen was diluted with a coating buffer (a PBS buffer, pH = 7.4) to prepare into an antigen to be coated with a concentration of 1 µg/mL. 100 µL of the antigen to be coated was added to a 96-well plate and incubated at room temperature for 1 hour. The PBS buffer (pH = 7.4) was used as a washing solution to remove unreacted antigens. 100 µL of a blocking solution was added to the 96-well plate and incubated at room temperature for 1 hour to block the surface of unreacted solid phases. Excess blocking solution was removed again with the washing solution (the PBS buffer, pH = 7.4). Then, 100 µL of a cell supernatant to be screened was added and incubated at room temperature for 30 minutes. An antibody that did not bind to the solid-phase-coated antigen was removed by washing with the washing solution (the PBS buffer, pH = 7.4), and then 100 µL of a peroxidase-labeled goat anti-mouse antibody was added. The peroxidase-labeled goat anti-mouse antibody binded to a mouse antibody recognizing the solid phase antigen, and catalyzed the luminescence of a substrate solution. The intensity of the luminescence was used to determine whether there was an antibody that might bind to the antigen in the cell supernatant.

During the antibody screening, the clone screened was confirmed to have no cross-reaction with sCD14 by using sCD14 for reverse screening. For a reverse screening experiment, a His-tag protein and an sCD14 protein were diluted with a coating buffer (a PBS buffer, pH = 7.4), respectively to prepare into a substance to be coated with a concentration of 1 µg/mL. The subsequent experimental steps were the same as that of the positive screening experiment. The intensity of the final luminescence value was used to determine whether the antibody in the cell supernatant was reactive with a reverse screening substance.

Subsequent subcloning was performed after screening of the parent clone. Affinity sorting and epitope classification were performed on a subclone. The subclone supernatant was paired with the polyclonal antibody prepared in Step 1.2 to detect the antigen. The results of affinity sorting and epitope classification of the anti-soluble CD14 subtype monoclonal antibody, and of screening of the paired antibody are summarized in Tables 5 to 7. According to the results of affinity sorting and antibody pairing, an antibody with the clone number of 16D5B2 was screened for subsequent production and purification, thereby obtaining a mouse monoclonal antibody.

**Table 5**

| Affinity sorting | | |
|---|---|---|
| | Clone number | EC50 (ng/ml) |
| 1 | 16D5B2 | 1.163 |
| 2 | 16D5B12 | 1.194 |
| 3 | 16D5D6 | 1.284 |
| 4 | 3D5D9 | 1.404 |
| 5 | 3D5A2 | 1.512 |
| 6 | 3D5D8 | 1.680 |
| 7 | 11H1E11 | 1.998 |
| 8 | 11H1B9 | 2.356 |
| 9 | 11H1G11 | 2.661 |
| 10 | 17C7A6 | 5.493 |
| 11 | 17C7H7 | 9.137 |
| 12 | 20A10F7 | / |
| 13 | 20A10E6 | / |

**Table 6**

| Epitope classification | |
|---|---|
| Epitope 1 | Epitope 2 |
| 3D5A2 | 17C7A6 |
| 3D5D8 | 17C7H7 |
| 3D5D9 | |
| 16D5B2 | |
| 16D5B12 | |
| 16D5D6 | |
| 11H1B9 | |
| 11H1E11 | |
| 11H1G11 | |

**Table 7**

| Screening of paired antibody | | | | |
|---|---|---|---|---|
| | Clone number | Antigen concentration | Polyclonal antibody Coating concentration | Corresponding value (RU) |
| 0 | Negative control (culture supernatant) | 100 nM | 5 µg/mL | -0.9 |
| 1 | 16D5B2 | | | 21.2 |
| 2 | 16D5B12 | | | 10.11 |
| 3 | 16D5D6 | | | 8.9 |
| 4 | 3D5D9 | | | 6.9 |
| 5 | 3D5A2 | | | 6.5 |
| 6 | 3D5D8 | | | 4.2 |
| 7 | 11H1E11 | | | -1.1 |
| 8 | 11H1B9 | | | 7.2 |
| 9 | 11H1G11 | | | -1.3 |
| 10 | 17C7A6 | | | 0.9 |
| 11 | 17C7H7 | | | 1.4 |

### Step 1.4: Determination of amino acid sequences of an anti-soluble CD14 subtype monoclonal antibody

The variable regions of the anti-soluble CD14 subtype monoclonal antibody obtained from screening in Step 1.3 were sequenced. First, the total RNA of hybridoma cells was extracted according to the instruction manual of TRIzol^{®} kit, and then the total RNA was reverse transcribed into cDNA using an epitope-specific antisense primer or universal primer according to the instruction manual of PrimeScript^{™} 1st Strand cDNA Synthesis Kit. The corresponding amino acid sequences of the anti-soluble CD14 subtype monoclonal antibody might be obtained by the reverse transcription-synthesized cDNA sequence. The anti-soluble CD14 subtype monoclonal antibody included, for example, VH comprising VH CDR1 composed of the amino acid sequence of SEQ ID NO: 3, VH CDR2 composed of the amino acid sequence of SEQ ID NO: 9 and VH CDR3 composed of the amino acid sequence of SEQ ID NO: 16, and VL comprising VL CDR1 composed of the amino acid sequence of SEQ ID NO: 21, VL CDR2 composed of the amino acid sequence of SEQ ID NO: 29 and VL CDR3 composed of the amino acid sequence of SEQ ID NO: 38, where the anti-soluble CD14 subtype monoclonal antibody specifically recognized an epitope composed of the amino acid sequence of SEQ ID No.42.

### Example 6: Determination of a specific epitope of an anti-soluble CD14 subtype monoclonal antibody

In the disclosure, a method for determining an epitope is not particularly defined, for example, it can be carried out in the following manner: firstly, a polypeptide was synthesized by successively moving 3 amino acids from the N-terminus to the C-terminus of the target protein sequence, and each peptide chain consisted of 15 amino acids; then, the polypeptide was labeled with biotin and coated on a 96-well plate containing streptavidin by biotin-streptavidin binding; next, an antibody to be detected and a peroxidase-labeled goat anti-mouse IgG were added to the 96-well plate; the amino acid sequences recognized by the antibody to be detected were determined by the signal value of the final reaction.

It can be seen from the various examples described above that the embodiments of the disclosure may detect a combined signal value of PCT and Presepsin in a sample in a reaction system, the comparison between the combined signal value and a reference value may be used for evaluating the possibility of a patient suffering from sepsis and evaluating the prognosis of a suspected sepsis patient, and compared with detecting PCT or Presepsin in a sample alone, a more efficient diagnosis and a better prognostic power are provided. In addition, for combined detection of PCT and Presepsin in a sample, only one detection is required, so that the economic cost and time cost of the detection may be reduced, the blood consumption may be decreased, and also the effect of reagents of different production batches on the detection results may be reduced, which is conducive to reagent production and instrument automation detection.

The disclosure is not limited to this by the description based on the embodiments. In particular, the disclosure includes each new feature and any combination of the features, especially any combination of features contained in the claims, even if the feature or the combination itself is not specified in detail in the claims or the embodiments.

## Claims

1. A kit for combined detection of procalcitonin and a soluble CD14 subtype in a sample to be tested, the kit comprising:
a capture antibody mixture, the capture antibody mixture comprising a procalcitonin capture antibody coated on a solid phase and a soluble CD14 subtype capture antibody coated on a solid phase; and
a detection antibody mixture, the detection antibody mixture comprising a labeled procalcitonin detection antibody and a labeled soluble CD14 subtype detection antibody;
wherein the procalcitonin detection antibody and the soluble CD14 subtype detection antibody are labeled with the same label.

2. The kit of claim 1, wherein the procalcitonin capture antibody and the soluble CD14 subtype capture antibody are separately coated on the solid phases.

3. The kit of claim 1 or 2, wherein a mixing ratio of the procalcitonin capture antibody to the soluble CD14 subtype capture antibody in the capture antibody mixture is in a range of 5: 1 to 1: 5; preferably in a range of 3: 1 to 1: 5, and more preferably in a range of 3: 1 to 1: 3.

4. The kit of claim 3, wherein the mixing ratio of the procalcitonin capture antibody to the soluble CD14 subtype capture antibody in the capture antibody mixture is 3: 1, 1: 1 or 1: 3, preferably 1: 3.

5. The kit of any one of claims 1 to 4, wherein a mixing ratio of the procalcitonin detection antibody to the soluble CD 14 subtype detection antibody in the detection antibody mixture is in a range of 5: 1 to 1: 5, preferably in a range of 3: 1 to 1:5, and more preferably in a range of 3: 1 to 1: 3.

6. The kit of claim 5, wherein the mixing ratio of the procalcitonin detection antibody to the soluble CD14 subtype detection antibody in the detection antibody mixture is 3: 1, 1: 1 or 1: 3, preferably 1: 3.

7. The kit of any one of claims 1 to 6, wherein a mixing ratio of the procalcitonin capture antibody to the soluble CD14 subtype capture antibody in the capture antibody mixture is equal to a mixing ratio of the procalcitonin detection antibody to the soluble CD14 subtype detection antibody in the detection antibody mixture, and the mixing ratios are preferably 1: 3.

8. The kit of any one of claims 1 to 7, wherein the soluble CD14 subtype capture antibody specifically recognizes an epitope composed of an amino acid sequence of SEQ ID No.42, and the soluble CD14 subtype capture antibody comprises:
(i) heavy chain variable region (VH) complementarity determining regions (CDRs):
VH CDR1: X₁X₂X₃MX₄;
VH CDR2: YIX₅X₆ADX₇;
VH CDR3: X₈X₉X₁₀AX₁₁;
and
(ii) light chain variable region (VL) complementarity determining regions (CDRs):
VL CDR1: KX₁₂X₁₃X₁₄N;
VL CDR2: LX₁₅X₁₆;
VL CDR3: VX₁₇X₁₈X₁₉;
where X₁ to X₁₉ are one or more of amino acid sequences listed below as options:
X₁=any one of amino acids;
X₂=F or V; X₃=A, E or K; X₄=A or L;
X₅=SYMGS, SSGSS, AYTMY, TYSGS or SSKSS;
X₆=GAYY, TIYY, AKYY, TKAS or SNLA;
X₇=AKLG, TVKG, SYTA, MTKG or YGNT;
X₈=A or Q; X₉=G or Q; X₁₀=Q or F; X₁₁=M, Y or V;
X₁₂=YYAS, SSAT, SSQS or SGSS;
X₁₃=AAKL, LLAT, LLYS or KAAS;
X₁₄=YRNIKL, TWAKGN, NGKTYL or YTNGAL;
X₁₅=VQS, KTS, QTS or VSK;
X₁₆=LAS, LDS, LTA or DSK;
X₁₇=G, A, S or Q;
X₁₈=GTH, GNT, GTA or TAI;
X₁₉=FITA, FPRT, YGHV or NYGH.

9. The kit of claim 8, wherein the soluble CD14 subtype capture antibody comprises:
(a) VH comprising VH CDR1 composed of an amino acid sequence of SEQ ID NO: 1, VH CDR2 composed of an amino acid sequence of SEQ ID NO: 12, and VH CDR3 composed of an amino acid sequence of SEQ ID NO: 17, and VL comprising VL CDR1 composed of an amino acid sequence of SEQ ID NO: 21, VL CDR2 composed of an amino acid sequence of SEQ ID NO: 32, and VL CDR3 composed of an amino acid sequence of SEQ ID NO: 33;
(b) VH comprising VH CDR1 composed of an amino acid sequence of SEQ ID NO: 2, VH CDR2 composed of an amino acid sequence of SEQ ID NO: 12, and VH CDR3 composed of an amino acid sequence of SEQ ID NO: 20, and VL comprising VL CDR1 composed of an amino acid sequence of SEQ ID NO: 22, VL CDR2 composed of an amino acid sequence of SEQ ID NO: 30, and VL CDR3 composed of an amino acid sequence of SEQ ID NO: 37;
(c) VH comprising VH CDR1 composed of an amino acid sequence of SEQ ID NO: 3, VH CDR2 composed of an amino acid sequence of SEQ ID NO: 9, and VH CDR3 composed of an amino acid sequence of SEQ ID NO: 16, and VL comprising VL CDR1 composed of an amino acid sequence of SEQ ID NO: 21, VL CDR2 composed of an amino acid sequence of SEQ ID NO: 29, and VL CDR3 composed of an amino acid sequence of SEQ ID NO: 38;
(d) VH comprising VH CDR1 composed of an amino acid sequence of SEQ ID NO: 5, VH CDR2 composed of an amino acid sequence of SEQ ID NO: 13, and VH CDR3 composed of an amino acid sequence of SEQ ID NO: 18, and VL comprising VL CDR1 composed of an amino acid sequence of SEQ ID NO: 21, VL CDR2 composed of an amino acid sequence of SEQ ID NO: 30, and VL CDR3 composed of an amino acid sequence of SEQ ID NO: 39;
(e) VH comprising VH CDR1 composed of an amino acid sequence of SEQ ID NO: 4, VH CDR2 composed of an amino acid sequence of SEQ ID NO: 12, and VH CDR3 composed of an amino acid sequence of SEQ ID NO: 16, and VL comprising VL CDR1 composed of an amino acid sequence of SEQ ID NO: 23, VL CDR2 composed of an amino acid sequence of SEQ ID NO: 30, and VL CDR3 composed of an amino acid sequence of SEQ ID NO: 33; or
(f) VH comprising VH CDR1 composed of an amino acid sequence of SEQ ID NO: 6, VH CDR2 composed of an amino acid sequence of SEQ ID NO: 13, and VH CDR3 composed of an amino acid sequence of SEQ ID NO: 20, and VL comprising VL CDR1 composed of an amino acid sequence of SEQ ID NO: 22, VL CDR2 composed of an amino acid sequence of SEQ ID NO: 30, and VL CDR3 composed of an amino acid sequence of SEQ ID NO: 38.

10. The kit of any one of claims 1 to 9,
wherein the soluble CD14 subtype capture antibody specifically recognizes the epitope composed of the amino acid sequence of SEQ ID No.42, or the soluble CD14 subtype capture antibody is an anti-soluble CD14 subtype monoclonal antibody or an antigen-binding antibody fragment thereof secreted and produced by a hybridoma cell strain with a deposit number of CGMCC NO.18536.

11. A method for combined detection of procalcitonin and a soluble CD14 subtype in a sample to be tested, the method comprising:
mixing a sample to be tested, a procalcitonin capture antibody coated on a solid phase, and a soluble CD14 subtype capture antibody coated on a solid phase, so that the procalcitonin capture antibody coated on the solid phase binds to the procalcitonin in the sample to be tested, and the soluble CD14 subtype capture antibody coated on the solid phase binds to the soluble CD14 subtype in the sample to be tested;
adding a labeled procalcitonin detection antibody and a labeled soluble CD14 subtype detection antibody to the mixture, so that the labeled procalcitonin detection antibody binds to the procalcitonin bound on the procalcitonin capture antibody to form a sandwich complex and the labeled soluble CD14 subtype detection antibody binds to the soluble CD14 subtype bound on the soluble CD14 subtype capture antibody to form a sandwich complex;
washing the sandwich complexes to remove unbound substances;
adding a detection substrate to the washed sandwich complexes to detect a combined concentration level of the procalcitonin and the soluble CD14 subtype in the sample to be tested;
wherein the procalcitonin detection antibody and the soluble CD14 subtype detection antibody has the same label, wherein the label is an enzyme, and the detection substrate is a substrate for the enzyme.

12. The method of claim 11, wherein a mixing ratio of the procalcitonin capture antibody to the soluble CD14 subtype capture antibody is in a range of 5: 1 to 1: 5, preferably in a range of 3: 1 to 1: 5, more preferably in a range of 3: 1 to 1: 3, and further more preferably is 1:3; or the procalcitonin detection antibody and the soluble CD14 subtype detection antibody are added to the mixture in a range of 5: 1 to 1: 5, preferably in a range of 3: 1 to 1: 5, more preferably in a range of 3: 1 to 1: 3, and further more preferably in a ratio of 1:3; or a mixing ratio of the procalcitonin capture antibody to the soluble CD14 subtype capture antibody is equal to a mixing ratio of the procalcitonin detection antibody to the soluble CD14 subtype detection antibody.

13. Use of a soluble CD14 subtype antibody and a procalcitonin antibody in the preparation of an analysis reagent for identifying whether a patient suffers from sepsis, wherein a sample to be tested from the patient, a procalcitonin capture antibody coated on a solid phase and a soluble CD14 subtype capture antibody coated on a solid phase are mixed, and then a labeled procalcitonin detection antibody and a labeled soluble CD14 subtype detection antibody are added to detect a combined concentration level of the procalcitonin and the soluble CD14 subtype in the sample to be tested, and an increase in the combined concentration level relative to a reference value is associated with an increase in possibility of the patient suffering from sepsis;
wherein the procalcitonin detection antibody and the soluble CD14 subtype detection antibody are labeled with the same label.

14. The use of claim 13, wherein the combined concentration value of the procalcitonin and the soluble CD14 subtype in the sample to be tested from the patient is detected within 72 hours after the patient is suspected of suffering from sepsis.

15. Use of a soluble CD14 subtype antibody and a procalcitonin antibody in the preparation of an analysis reagent for evaluating the prognosis of a suspected sepsis patient, wherein a sample to be tested from the patient, a procalcitonin capture antibody coated on a solid phase and a soluble CD14 subtype capture antibody coated on a solid phase are mixed, and then a labeled procalcitonin detection antibody and a labeled soluble CD 14 subtype detection antibody are added to detect a combined concentration level of the procalcitonin and the soluble CD14 subtype in the sample to be tested, and an increase in the combined concentration level relative to a reference value is associated with an increase in risk of death in the suspected sepsis patient;
wherein the procalcitonin detection antibody and the soluble CD14 subtype detection antibody are labeled with the same label.

## Patentansprüche

1. Kit zum kombinierten Nachweis von Procalcitonin und einem löslichen CD-14-Subtyp in einer zu untersuchenden Probe, wobei das Kit Folgendes umfasst:
eine Fangantikörpermischung, wobei diese einen auf eine Festphase aufgetragenen Procalcitonin-Fangantikörper und einen auf eine Festphase aufgetragenen löslichen CD-14-Subtyp-Fangantikörper umfasst; und
eine Antikörpermischung zum Nachweis, wobei diese einen markierten Procalcitonin-Nachweisantikörper und einen markierten löslichen CD-14-Subtyp-Nachweisantikörper umfasst;
wobei der Antikörper zum Nachweis von Procalcitonin und der Antikörper zum Nachweis des löslichen CD-14-Subtyps mit derselben Markierung versehen sind.

2. Kit nach Anspruch 1, wobei der Procalcitonin-Fangantikörper und der lösliche CD-14-Subtyp-Fangantikörper getrennt auf die Festphasen aufgetragen sind.

3. Kit nach Anspruch 1 oder 2, wobei das Mischungsverhältnis des Procalcitonin-Fangantikörpers zu dem löslichen CD-14-Subtyp-Fangantikörper in der Fangantikörper-Mischung im Bereich von 5: 1 bis 1: 5, vorzugsweise im Bereich von 3: 1 bis 1: 5 und noch bevorzugter im Bereich von 3: 1 bis 1: 3 liegt.

4. Kit nach Anspruch 3, wobei das Mischungsverhältnis des Fangantikörpers gegen Procalcitonin zu dem löslichen CD-14-Subtyp-Antikörper in der nachweisenden Antikörpermischung 3: 1, 1: 1 oder 1: 3, vorzugsweise 1: 3, beträgt.

5. Kit nach einem der Ansprüche 1 bis 4, wobei das Mischungsverhältnis des nachweisenden Procalcitonin-Antikörpers zu dem nachweisenden löslichen CD-14-Subtyp-Antikörper in der nachweisenden Antikörpermischung in einem Bereich von 5: 1 bis 1: 5, vorzugsweise in einem Bereich von 3: 1 bis 1:5 und besonders bevorzugt in einem Bereich von 3: 1 bis 1: 3 liegt.

6. Kit nach Anspruch 5, wobei das Mischungsverhältnis des nachweisenden Procalcitonin-Antikörpers zum nachweisenden löslichen CD-14-Subtyp-Antikörper in der nachweisenden Antikörpermischung 3: 1, 1: 1 oder 1: 3, vorzugsweise 1:3, beträgt.

7. Kit nach einem der Ansprüche 1 bis 6, wobei das Mischungsverhältnis des Procalcitonin-Fangantikörpers zum löslichen CD-14-Subtyp-Fangantikörper in der Fangantikörpermischung gleich dem Mischungsverhältnis des Procalcitonin-nachweisenden Antikörpers zum löslichen CD-14-Subtyp-nachweisenden Antikörper in der nachweisenden Antikörpermischung ist, und die Mischungsverhältnisse vorzugsweise 1:3 sind.

8. Kit nach einem der Ansprüche 1 bis 7, wobei der lösliche CD-14-Subtyp-Fangantikörper spezifisch ein Epitop erkennt, welches aus einer Aminosäuresequenz der SEQ ID Nr. 42 besteht, und der lösliche CD-14-Subtyp-Fangantikörper Folgendes aufweist:
(i) Komplementarität-bestimmend Regionen (CDRs) der variablen Region auf der schweren Kette (VH):
VH CDR1: X₁X₂X₃MX₄; VH
CDR2: YIX₅X₆ADX₇; VH CDR3:
X₈X₉X₁₀AX_{II};
und
(ii) Komplementarität-bestimmend Regionen (CDRs) der variablen Region auf der leichten Kette (VH)
VL CDR1: KX₁₂X₁₃X₁₄N;
VL CDR2: LX₁₅X₁₆ ;
VL CDR3: VX₁₇X₁₈X₁₉ ;
wobei Xi bis X19 eine oder mehrere der nachstehend als Optionen aufgeführten Aminosäuresequenzen sind:
X₁= eine der Aminosäuren;
X₂=F oder V; X₃=A, E oder K; X₄=A oder L;
X₅=SYMGS, SSGSS, AYTMY, TYSGS oder SSKSS;
X₆=GAYY, TIYY, AKYY, TKAS oder SNLA;
X₇=AKLG, TVKG, SYTA, MTKG oder YGNT;
X₈=A or Q; X₉=G or Q; X oder F; Xn=M, Y or V;
X₁₂ =YYAS, SSAT, SSQS oder SGSS;
X₁₃=AAKL, LLAT, LLYS oder KAAS;
X₁₄=YRNIKL, TWAKGN, NGKTYL oder YTNGAL;
X₁₅=VQS, KTS, QTS oder VSK;
X₁₆=LAS, LDS, LTA oder DSK;
X₁₇=G, A, S oder Q;
X₁₈=GTH, GNT, GTA oder TAI;
X_{I9}=FITA, FPRT, YGHV oder NYGH.

9. Kit nach Anspruch 8, wobei der lösliche CD-14-Subtyp-Fangantikörper Folgendes umfasst:
(a) VH mit VH CDR1, bestehend aus einer Aminosäuresequenz der SEQ ID NO: 1, VH CDR2 bestehend aus einer Aminosäuresequenz der SEQ ID NO: 12, und VH CDR3 bestehend aus einer Aminosäuresequenz der SEQ ID NO: 17, und VL mit VL CDR1 bestehend aus einer Aminosäuresequenz der SEQ ID NO: 21, VL CDR2 bestehend aus einer Aminosäuresequenz der SEQ ID NO: 32, und VL CDR3 bestehend aus einer Aminosäuresequenz der SEQ ID NO: 33;
(b) VH mit VH CDR1 bestehend aus einer Aminosäuresequenz der SEQ ID NO: 2, VH CDR2 bestehend aus einer Aminosäuresequenz der SEQ ID NO: 12, und VH CDR3 bestehend aus einer Aminosäuresequenz der SEQ ID NO: 20, und VL mit VL CDR1 bestehend aus einer Aminosäuresequenz der SEQ ID NO: 22, VL CDR2 bestehend aus einer Aminosäuresequenz der SEQ ID NO: 30, und VL CDR3 bestehend aus einer Aminosäuresequenz der SEQ ID NO: 37;
(c) VH mit VH CDR1 bestehend aus einer Aminosäuresequenz der SEQ ID NO: 3, VH CDR2 bestehend aus einer Aminosäuresequenz der SEQ ID NO: 9, und VH CDR3 bestehend aus einer Aminosäuresequenz der SEQ ID NO: 16, und VL mit VL CDR1 bestehend aus einer Aminosäuresequenz der SEQ ID NO: 21, VL CDR2 bestehend aus einer Aminosäuresequenz der SEQ ID NO: 29, und VL CDR3 bestehend aus einer Aminosäuresequenz der SEQ ID NO: 38;
(d) VH mit VH CDR1 bestehend aus einer Aminosäuresequenz der SEQ ID NO: 5, VH CDR2 bestehend aus einer Aminosäuresequenz der SEQ ID NO: 13, und VH CDR3 bestehend aus einer Aminosäuresequenz der SEQ ID NO: 18, und VL mit VL CDR1 bestehend aus einer Aminosäuresequenz der SEQ ID NO: 21, VL CDR2 bestehend aus einer Aminosäuresequenz der SEQ ID NO: 30, und VL CDR3 bestehend aus einer Aminosäuresequenz der SEQ ID NO: 39;
(e) VH mit VH CDR1 bestehend aus einer Aminosäuresequenz der SEQ ID NO: 4, VH CDR2 bestehend aus einer Aminosäuresequenz der SEQ ID NO: 12, und VH CDR3 bestehend aus einer Aminosäuresequenz der SEQ ID NO: 16, und VL mit VL CDR1 bestehend aus einer Aminosäuresequenz der SEQ ID NO: 23, VL CDR2 bestehend aus einer Aminosäuresequenz der SEQ ID NO: 30, und VL CDR3 bestehend aus einer Aminosäuresequenz der SEQ ID NO: 33; oder
(f) VH mit VH CDR1 bestehend aus einer Aminosäuresequenz der SEQ ID NO: 6, VH CDR2 bestehend aus einer Aminosäuresequenz der SEQ ID NO: 13, und VH CDR3 bestehend aus einer Aminosäuresequenz der SEQ ID NO: 20, und VL mit VL CDR1 bestehend aus einer Aminosäuresequenz der SEQ ID NO: 22, VL CDR2 bestehend aus einer Aminosäuresequenz der SEQ ID NO: 30, und VL CDR3 bestehend aus einer Aminosäuresequenz der SEQ ID NO: 38.

10. Kit nach einem der Ansprüche 1 bis 9, wobei der lösliche CD-14-Subtyp-Fangantikörper insbesondere das Epitop erkennt, das aus der Aminosäuresequenz der SEQ ID Nr. 42 besteht, oder der lösliche CD-14-Subtyp-Fangantikörper ein monoklonaler Antikörper gegen den löslichen CD-14-Subtyp oder ein Antigen-bindendes Antikörperfragment davon ist, das von einem Hybridom-Zellstamm mit der Hinterlegungsnummer CGMCC Nr. 18536 sezerniert und produziert wird.

11. Verfahren zum kombinierten Nachweis von Procalcitonin und einem löslichen CD-14-Subtyp in einer zu untersuchenden Probe, wobei das Verfahren Folgendes umfasst:
Mischen einer zu untersuchenden Probe, eines auf eine Festphase aufgetragenen Procalcitonin-Fangantikörpers und eines auf eine Festphase aufgetragenen löslichen CD-14-Subtyp-Fangantikörpers, so dass der auf die Festphase aufgetragene Procalcitonin-Fangantikörper an das Procalcitonin in der zu untersuchenden Probe bindet, und der auf die Festphase aufgetragene lösliche CD-14-Subtyp-Fangantikörper an den löslichen CD-14-Subtyp in der zu untersuchenden Probe bindet;
Zufügen eines markierten nachweisenden Procalcitonin-Antikörpers und eines markierten nachweisenden Antikörpers für den löslichen CD-14-Subtyp zu der Mischung, so dass der markierte nachweisende Procalcitonin-Antikörper an das an den Procalcitonin-Fangantikörper gebundene Procalcitonin bindet, um einen sogenannten Sandwichkomplex zu bilden, und der markierte nachweisende Antikörper für den löslichen CD-14-Subtyp an den an den löslichen CD-14-Subtyp-Fangantikörper gebundenen löslichen CD-14-Subtyp bindet, um einen Sandwichkomplex zu bilden;
Auswaschen der Sandwichkomplexe zur Entfernung nichtgebundener Substanzen;
Hinzufügen eines Nachweissubstrats zu den ausgewaschenen Sandwichkomplexen, um eine kombinierte Konzentrationsmenge des Procalcitonins und des löslichen CD-14-Subtyps in der zu testenden Probe nachzuweisen;
wobei der nachweisende Procalcitonin-Antikörper und der nachweisende lösliche CD-14-Subtyp-Antikörper die gleiche Markierung aufweisen, wobei die Markierung ein Enzym ist und das nachweisende Substrat ein Substrat für das Enzym ist.

12. Verfahren nach Anspruch 11, wobei das Mischungsverhältnis des Procalcitonin-Fangantikörpers zu dem löslichen CD-14-Subtyp-Fangantikörper im Bereich von 5: 1 bis 1: 5, vorzugsweise im Bereich von 3: 1 bis 1: 5, besonders bevorzugt im Bereich von 3: 1 bis 1: 3 und noch bevorzugter im Bereich von 1:3 liegt; oder der nachweisende Procalcitonin-Antikörper und der nachweisende lösliche CD-14-Subtyp-Antikörper werden der Mischung in einem Verhältnis von 5:1 bis 1:5, vorzugsweise in einem Verhältnis von 3:1 bis 1:5, besonders bevorzugt in einem Verhältnis von 3:1 bis 1:3 und noch bevorzugter in einem Verhältnis von 1:3 zugesetzt; oder das Mischungsverhältnis des Procalcitonin-Fangantikörpers zu dem löslichen CD-14-Subtyp-Fangantikörper ist gleich dem Mischungsverhältnis des nachweisenden Procalcitonin-Antikörpers zu dem nachweisenden löslichen CD-14-Subtyp-Antikörper.

13. Verwendung eines löslichen CD-14-Subtyp-Antikörpers und eines Procalcitonin-Antikörpers bei der Herstellung eines Analysereagens zur Bestimmung, ob ein Patient an Sepsis leidet, wobei eine zu testende Probe des Patienten, ein auf eine Festphase beschichteter Procalcitonin-Fangantikörper und ein auf eine Festphase beschichteter löslicher CD-14-Subtyp-Fangantikörper gemischt werden, und dann ein markierter nachweisender Procalcitonin-Antikörper und ein markierter nachweisender Antikörper für den löslichen CD-14-Subtyp zugegeben werden, um einen kombinierten Konzentrationswert des Procalcitonins und des löslichen CD-14-Subtyps in der zu testenden Probe nachzuweisen, und ein Anstieg des kombinierten Konzentrationswertes verglichen mit einem Referenzwert mit zunehmender Wahrscheinlichkeit verbunden ist, dass der Patient an Sepsis leidet.;
wobei der Antikörper zum Nachweis von Procalcitonin und der Antikörper zum Nachweis des löslichen CD-14-Subtyps mit derselben Markierung versehen sind.

14. Verwendung nach Anspruch 13, wobei der kombinierte Konzentrationswert des Procalcitonins und des löslichen CD 14-Subtyps in der zu untersuchenden Probe des Patienten innerhalb von 72 Stunden, nachdem bei dem Patienten der Verdacht auf Sepsis besteht, nachgewiesen wird.

15. Verwendung eines löslichen CD-14-Subtyp-Antikörpers und eines Procalcitonin-Antikörpers bei der Herstellung eines Analysereagens zur Bewertung der Prognose eines Patienten mit Verdacht auf Sepsis, wobei eine zu testende Probe des Patienten, ein auf eine Festphase aufgetragener Procalcitonin-Fangantikörper und ein auf eine Festphase aufgetragener löslicher CD-14-Subtyp-Fangantikörper gemischt werden, und dann ein markierter nachweisender Procalcitonin-Antikörper und ein markierter nachweisender Antikörper für den löslichen CD-14-Subtyp zugegeben werden, um einen kombinierten Konzentrationswert des Procalcitonins und des löslichen CD-14-Subtyps in der zu testenden Probe nachzuweisen, und ein Anstieg des kombinierten Konzentrationswertes im Vergleich zu einem Referenzwert mit einem erhöhten Sterberisiko bei dem Patienten mit Sepsisverdacht verbunden ist;
wobei der Antikörper zum Nachweis von Procalcitonin und der Antikörper zum Nachweis des löslichen CD-14-Subtyps mit derselben Markierung versehen sind.

## Revendications

1. Kit de détection combinée de procalcitonine et d'un sous-type CD14 soluble dans un échantillon à analyser, le kit comprenant :
un mélange d'anticorps de capture, le mélange d'anticorps de capture comprenant un anticorps de capture de procalcitonine enduit sur une phase solide et un anticorps de capture de sous-type CD14 soluble enduit sur une phase solide ; et
un mélange d'anticorps de détection, le mélange d'anticorps de détection comprenant un anticorps de détection de procalcitonine marqué et un anticorps de détection de sous-type CD14 soluble marqué ;
où l'anticorps de détection de procalcitonine et l'anticorps de détection de sous-type CD14 soluble sont marqués avec le même marqueur.

2. Kit selon la revendication 1, dans lequel l'anticorps de capture de procalcitonine et l'anticorps de capture de sous-type CD14 soluble sont enduits séparément sur les phases solides.

3. Kit selon la revendication 1 ou 2, dans lequel un ratio de mélange de l'anticorps de capture de procalcitonine sur l'anticorps de capture de sous-type CD14 soluble dans le mélange d'anticorps de capture se situe dans une plage allant de 5:1 à 1:5, de préférence dans une plage allant de 3:1 à 1:5, et de façon davantage préférée dans une plage allant de 3:1 à 1:3.

4. Kit selon la revendication 3, dans lequel le ratio de mélange de l'anticorps de capture de procalcitonine sur l'anticorps de capture de sous-type CD14 soluble dans le mélange d'anticorps de capture est de 3:1, de 1:1 ou de 1:3, de préférence de 1:3.

5. Kit selon l'une quelconque des revendications 1 à 4, dans lequel un ratio de mélange de l'anticorps de détection de procalcitonine sur l'anticorps de détection de sous-type CD14 soluble dans le mélange d'anticorps de détection se situe dans une plage allant de 5:1 à 1:5, de préférence dans une plage allant de 3:1 à 1:5, et de façon davantage préférée dans une plage allant de 3:1 à 1:3.

6. Kit selon la revendication 5, dans lequel le ratio de mélange de l'anticorps de détection de procalcitonine sur l'anticorps de détection de sous-type CD14 soluble dans le mélange d'anticorps de détection est de 3:1, de 1:1 ou de 1:3, de préférence de 1:3.

7. Kit selon l'une quelconque des revendications 1 à 6, dans lequel un ratio de mélange de l'anticorps de capture de procalcitonine sur l'anticorps de capture de sous-type CD14 soluble dans le mélange d'anticorps de capture est égal à un ratio de mélange de l'anticorps de détection de procalcitonine sur l'anticorps de détection de sous-type CD14 soluble dans le mélange d'anticorps de détection, et les ratios de mélange sont de préférence de 1:3.

8. Kit selon l'une quelconque des revendications 1 à 7, dans lequel l'anticorps de capture de sous-type CD14 soluble reconnaît spécifiquement un épitope composé d'une séquence d'acides aminés à numéro d'identification SEQ ID N° 42, et l'anticorps de capture de sous-type CD14 soluble comprend :
(i) des régions de détermination de complémentarité (CDR) de région variable à chaîne lourde (VH) :
VH CDR1 : X₁X₂X₃MX₄ ;
VH CDR2 : YIX₅X₆X₃ADX₇ ;
VH CDR3 : X₈X₉X₁₀AX₁₁ ;
et
(ii) des régions de détermination de complémentarité (CDR) de région variable à chaîne légère (VL) :
VL CDR1 : KX₁₂X₁₃X₁₄N ;
VL CDR2 : LX₁₅X₁₆ ;
VL CDR3 : VX₁₇X₁₈X₁₉ ;
où X1 à X19 sont une ou plusieurs des séquences d'acides aminés répertoriées ci-dessous en tant qu'options :
X₁ = l'un quelconque des acides aminés ;
X₂ = F ou V; X₃ = A, E ou K; X₄ = A ou L ;
X₅ = SYMGS, SSGSS, AYTMY, TYSGS ou SSKSS ;
X₆ = GAYY, TIYY, AKYY, TKAS ou SNLA ;
X₇ = AKLG, TVKG, SYTA, MTKG ou YGNT ;
X₈ = A ou Q; X₉ = G ou Q; X₁₀ = Q ou F; X₁₁ = M, Y ou V ;
X₁₂ = YYAS, SSAT, SSQS ou SGSS ;
X₁₃ = AAKL, LLAT, LLYS ou KAAS ;
X₁₄ = YRNIKL, TWAKGN, NGKTYL ou YTNGAL ;
X₁₅ = VQS, KTS, QTS ou VSK ;
X₁₆ = LAS, LDS, LTA ou DSK ;
X₁₇ = G, A, S ou Q ;
X₁₈ = GTH, GNT, GTA ou TAI ;
X₁₉ = FITA, FPRT, YGHV ou NYGH.

9. Kit selon la revendication 8, dans lequel l'anticorps de capture de sous-type CD14 soluble comprend :
(a) VH comprenant VH CDR1 composé d'une séquence d'acides aminés à numéro d'identification SEQ ID N° 1, VH CDR2 composé d'une séquence d'acides aminés à numéro d'identification SEQ ID N° 12, et VH CDR3 composé d'une séquence d'acides aminés à numéro d'identification SEQ ID N° 17, et VL comprenant VL CDR1 composé d'une séquence d'acides aminés à numéro d'identification SEQ ID N° 21, VL CDR2 composé d'une séquence d'acides aminés à numéro d'identification SEQ ID N° 32, et VL CDR3 composé d'une séquence d'acides aminés à numéro d'identification SEQ ID N° 33 ;
(b) VH comprenant VH CDR1 composé d'une séquence d'acides aminés à numéro d'identification SEQ ID N° 2, VH CDR2 composé d'une séquence d'acides aminés à numéro d'identification SEQ ID N° 12, et VH CDR3 composé d'une séquence d'acides aminés à numéro d'identification SEQ ID N° 20, et VL comprenant VL CDR1 composé d'une séquence d'acides aminés à numéro d'identification SEQ ID N° 22, VL CDR2 composé d'une séquence d'acides aminés à numéro d'identification SEQ ID N° 30, et VL CDR3 composé d'une séquence d'acides aminés à numéro d'identification SEQ ID N° 37 ;
(c) VH comprenant VH CDR1 composé d'une séquence d'acides aminés à numéro d'identification SEQ ID N° 3, VH CDR2 composé d'une séquence d'acides aminés à numéro d'identification SEQ ID N° 9, et VH CDR3 composé d'une séquence d'acides aminés à numéro d'identification SEQ ID N° 16, et VL comprenant VL CDR1 composé d'une séquence d'acides aminés à numéro d'identification SEQ ID N° 21, VL CDR2 composé d'une séquence d'acides aminés à numéro d'identification SEQ ID N° 29, et VL CDR3 composé d'une séquence d'acides aminés à numéro d'identification SEQ ID N° 38 ;
(d) VH comprenant VH CDR1 composé d'une séquence d'acides aminés à numéro d'identification SEQ ID N° 5, VH CDR2 composé d'une séquence d'acides aminés à numéro d'identification SEQ ID N° 13, et VH CDR3 composé d'une séquence d'acides aminés à numéro d'identification SEQ ID N° 18, et VL comprenant VL CDR1 composé d'une séquence d'acides aminés à numéro d'identification SEQ ID N° 21, VL CDR2 composé d'une séquence d'acides aminés à numéro d'identification SEQ ID N° 30, et VL CDR3 composé d'une séquence d'acides aminés à numéro d'identification SEQ ID N° 39 ;
(e) VH comprenant VH CDR1 composé d'une séquence d'acides aminés à numéro d'identification SEQ ID N° 4, VH CDR2 composé d'une séquence d'acides aminés à numéro d'identification SEQ ID N° 12, et VH CDR3 composé d'une séquence d'acides aminés à numéro d'identification SEQ ID N° 16, et VL comprenant VL CDR1 composé d'une séquence d'acides aminés à numéro d'identification SEQ ID N° 23, VL CDR2 composé d'une séquence d'acides aminés à numéro d'identification SEQ ID N° 30, et VL CDR3 composé d'une séquence d'acides aminés à numéro d'identification SEQ ID N° 33 ; ou
(f) VH comprenant VH CDR1 composé d'une séquence d'acides aminés à numéro d'identification SEQ ID N° 6, VH CDR2 composé d'une séquence d'acides aminés à numéro d'identification SEQ ID N° 13, et VH CDR3 composé d'une séquence d'acides aminés à numéro d'identification SEQ ID N° 20, et VL comprenant VL CDR1 composé d'une séquence d'acides aminés à numéro d'identification SEQ ID N° 22, VL CDR2 composé d'une séquence d'acides aminés à numéro d'identification SEQ ID N° 30, et VL CDR3 composé d'une séquence d'acides aminés à numéro d'identification SEQ ID N° 38.

10. Kit selon l'une quelconque des revendications 1 à 9,
dans lequel l'anticorps de capture de sous-type CD14 soluble reconnaît spécifiquement l'épitope composé de la séquence d'acides aminés à numéro d'identification SEQ ID N° 42, ou l'anticorps de capture de sous-type CD14 soluble est un anticorps monoclonal anti-sous-type CD14 soluble ou un fragment d'anticorps de liaison d'antigène de celui-ci sécrété et produit par une souche cellulaire d'hybridome ayant pour numéro d'enregistrement CGMCC N° 18536.

11. Procédé de détection combinée de procalcitonine et d'un sous-type CD14 soluble dans un échantillon à analyser, le procédé comprenant les étapes consistant à :
mélanger un échantillon à analyser, un anticorps de capture de procalcitonine enduit sur une phase solide et un anticorps de capture de sous-type CD14 soluble enduit sur une phase solide, de sorte que l'anticorps de capture de procalcitonine enduit sur la phase solide se lie à la procalcitonine dans l'échantillon à analyser, et que l'anticorps de capture de sous-type CD14 soluble enduit sur la phase solide se lie au sous-type CD14 soluble dans l'échantillon à analyser ;
ajouter un anticorps de détection de procalcitonine marqué et un anticorps de détection de sous-type CD14 soluble marqué au mélange, de sorte que l'anticorps de détection de procalcitonine marqué se lie à la procalcitonine liée à l'anticorps de capture de procalcitonine pour former un complexe en sandwich et que l'anticorps de détection de sous-type CD14 soluble marqué se lie au sous-type CD14 soluble lié à l'anticorps de capture de sous-type CD14 soluble pour former un complexe en sandwich ;
laver les complexes en sandwich pour éliminer des substances non liées ;
ajouter un substrat de détection aux complexes en sandwich lavés pour détecter un niveau de concentration combiné de la procalcitonine et du sous-type CD14 soluble dans l'échantillon à analyser ;
où l'anticorps de détection de procalcitonine et l'anticorps de détection de sous-type CD14 soluble ont le même marqueur, où le marqueur est une enzyme, et le substrat de détection est un substrat destiné à l'enzyme.

12. Procédé selon la revendication 11, dans lequel un ratio de mélange de l'anticorps de capture de procalcitonine sur l'anticorps de capture de sous-type CD14 soluble se situe dans une plage allant de 5:1 à 1:5, de préférence dans une plage allant de 3:1 à 1:5, de façon davantage préférée dans une plage allant de 3:1 à 1:3, et de manière encore plus préférée est de 1:3 ; ou l'anticorps de détection de procalcitonine et l'anticorps de détection de sous-type CD14 soluble sont ajoutés au mélange dans une plage allant de 5:1 à 1:5, de préférence dans une plage allant de 3:1 à 1:5, de façon davantage préférée dans une plage allant de 3:1 à 1:3, et de manière encore plus préférée selon un ratio de 1:3 ; ou un ratio de mélange de l'anticorps de capture de procalcitonine sur l'anticorps de capture de sous-type CD14 soluble est égal à un ratio de mélange de l'anticorps de détection de procalcitonine sur l'anticorps de détection de sous-type CD14 soluble.

13. Utilisation d'un anticorps de sous-type CD14 soluble et d'un anticorps de procalcitonine dans la préparation d'un réactif d'analyse pour identifier si un patient souffre de septicémie, où un échantillon à analyser provenant du patient, un anticorps de capture de procalcitonine enduit sur une phase solide et un anticorps de capture de sous-type CD14 soluble enduit sur une phase solide sont mélangés, et ensuite un anticorps de détection de procalcitonine marqué et un anticorps de détection de sous-type CD14 soluble marqué sont ajoutés pour détecter un niveau de concentration combiné de la procalcitonine et du sous-type CD14 soluble dans l'échantillon à analyser, et une augmentation du niveau de concentration combiné par rapport à une valeur de référence est associée à une augmentation de la possibilité que le patient souffre de septicémie ;
où l'anticorps de détection de procalcitonine et l'anticorps de détection de sous-type CD14 soluble sont marqués avec le même marqueur.

14. Utilisation selon la revendication 13, où la valeur de concentration combinée de la procalcitonine et du sous-type CD14 soluble dans l'échantillon à analyser provenant du patient est détectée dans les 72 heures qui suivent l'instant où le patient est suspecté de souffrir de septicémie.

15. Utilisation d'un anticorps de sous-type CD14 soluble et d'un anticorps de procalcitonine dans la préparation d'un réactif d'analyse pour évaluer le pronostic d'un patient suspecté de souffrir de septicémie, où un échantillon à analyser provenant du patient, un anticorps de capture de procalcitonine enduit sur une phase solide et un anticorps de capture de sous-type CD14 soluble enduit sur une phase solide sont mélangés, et ensuite un anticorps de détection de procalcitonine marqué et un anticorps de détection de sous-type CD14 soluble marqué sont ajoutés pour détecter un niveau de concentration combiné de la procalcitonine et du sous-type CD14 soluble dans l'échantillon à analyser, et une augmentation du niveau de concentration combiné par rapport à une valeur de référence est associée à une augmentation du risque de décès du patient suspecté de souffrir de septicémie ;
où l'anticorps de détection de procalcitonine et l'anticorps de détection de sous-type CD14 soluble sont marqués avec le même marqueur.
